(19) Europäisches Patentamt
European Patent Office
Office européen des brevets

(11) EP 1 910 299 B1

(12) **EUROPEAN PATENT SPECIFICATION**

(45) Date of publication and mention of the grant of the patent:
**07.09.2011 Bulletin 2011/36**

(21) Application number: **06762612.7**

(22) Date of filing: **15.07.2006**

(51) Int Cl.:
***C07D 231/06*** *(2006.01)* ***A61K 31/4155*** *(2006.01)*
***A61P 25/00*** *(2006.01)*

(86) International application number:
**PCT/EP2006/006969**

(87) International publication number:
**WO 2007/009695 (25.01.2007 Gazette 2007/04)**

(54) **SUBSTITUTED PYRAZOLINE COMPOUNDS, THEIR PREPARATION AND USE AS MEDICAMENTS**

SUBSTITUIERTE PYRAZOLINVERBINDUNGEN, DEREN HERSTELLUNG UND VERWENDUNG ALS MEDIKAMENTE

COMPOSES PYRAZOLINIQUES SUBSTITUES, ELABORATION ET UTILISATION MEDICAMENTEUSE

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.07.2005 EP 05384005**
**05.08.2005 US 705481 P**

(43) Date of publication of application:
**16.04.2008 Bulletin 2008/16**

(73) Proprietor: **Laboratorios del Dr. Esteve S.A.**
**08041 Barcelona (ES)**

(72) Inventor: **CUBERES ALTISEN, Maria Rosa**
**Barcelona (ES)**

(74) Representative: **Peters, Hajo et al**
**ZACCO GmbH**
**Bayerstrasse 83**
**80335 München (DE)**

(56) References cited:
**EP-A- 1 083 171** **WO-A-02/076949**
**WO-A-2005/077911** **US-A1- 2005 096 373**

- **SHIM J-Y ET AL: "Molecular interaction of the antagonist N-(piperidin-1-yl)-5-(4-chlor ophenyl)-1-(2,4-dichlorophenyl)-4-methyl-1 H-pyrazole-3-carboxamide with the CB1 cannabinoid receptor" JOURNAL OF MEDICINAL CHEMISTRY, AMERICAN CHEMICAL SOCIETY. WASHINGTON, US, vol. 45, no. 7, March 2002 (2002-03), pages 1447-1459, XP002968557 ISSN: 0022-2623**

Remarks:
The file contains technical information submitted after the application was filed and not included in this specification

EP 1 910 299 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to substituted pyrazoline compounds, methods for their preparation, medicaments comprising these compounds as well as their use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occurring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of Cannabinoid receptors, more particularly for the modulation of Cannabinoid 1 ($CB_1$) receptors.

Said object was achieved by providing the substituted pyrazoline compounds of general formula I given below, their stereoisomers, corresponding salts and corresponding solvates thereof.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to substituted pyrazoline compounds of general formula **I**,

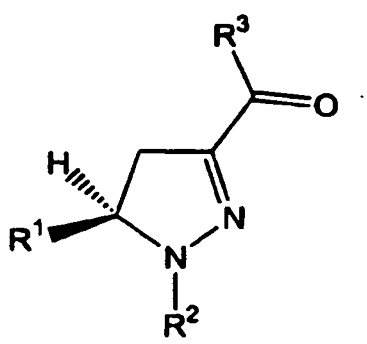

**I**

wherein

$R^1$ represents a phenyl group, which is optionally substituted by one or more substituents independently selected from the group consisting of methyl, ethyl, F, Cl, Br and $CF_3$.

$R^2$ represents a phenyl group, which is optionally substituted by one or more substituents independently selected from the group consisting of methyl, ethyl, F, Cl, Br and $CF_3$,

$R^3$ represents a pyrrolidinyl group, a piperidinyl group or a piperazinyl group, whereby each of these groups may be substituted with one or more $C_{1-6}$-alkyl groups, or $R^3$ represents an $-NR^4R^5$-moiety,

one of the residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an $-SO_2-R^6$-moiety; or an $-NR^7R^8$-moiety, or $R^4$ and $R^5$, identical or different, represent a methyl group, an ethyl group, an n-propyl group, an isopropyl group, an n-butyl group, a sec-butyl group or a tert.-butyl group,

$R^6$ represents a $C_{1-6}$alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups, and

$R^7$ and $R^8$, identical or different, represent a hydrogen atom or a $C_{1-6}$ alkyl radical

optionally in form of a corresponding N-oxide thereof, or a corresponding salt thereof, or a corresponding solvate thereof.

A mono- or polycyclic ring-system according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

The term "condensed" according to the present invention means that a ring or ring-system is attached to another ring or ring-system, whereby the terms "annulated" or "annelated" are also used by those skilled in the art to designate this kind of attachment.

If one or more of the residues $R^3$-$R^8$ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, oxo, amino, carboxy, amido, cyano, nitro, $-SO_2NH_2$, $-CO-C_{1-4}$-alkyl, $-SO-C_{1-4}$-alkyl, $-SO_2-C_{1-4}$-alkyl, $-NH-SO_2-C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, oxo, $CF_3$ and a phenyl group.

If one or more of the residues $R^3$-$R^8$ represents or comprises a cycloaliphatic group, which contains one or more heteroatoms as ring members, unless defined otherwise, each of these heteroatoms may preferably be selected from the group consisting of N, O and S. Preferably a cycloaliphatic group may contain 1, 2 or 3 heteroatoms independently selected from the group consisting of N, O and S as ring members. Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl, homo-Piperazinyl and Morpholinyl.

If one or more of the residues $R^3$-$R^8$ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, amino, carboxy, oxo, amido, cyano, nitro, $-SO_2NH_2$, $-CO-C_{1-4}$-alkyl, $-SO-C_{1-4}$-alkyl, $-SO_2-C_{1-4}$-alkyl, $-NH-SO_2-C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, $CF_3$, oxo and a phenyl group.

If one or more of the residues $R^1$-$R^8$ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (e.g. F, Cl. Br, I), a linear or branched $C_{1-6}$-alkyl group, a linear or branched $C_{1-6}$ alkoxy group, a formyl group, a hydroxy group, a trifluoromethyl group, a trifluoromethoxy group, a $-CO-C_{1-6}$-alkyl group, a cyano group, a nitro group, a carboxy group, a $-CO-O-C_{1-6}$-alkyl group, a $-CO-NR^AR^B$-moiety, a $-CO-NH-NR^CR^D$-moiety, an -SH, an $-S-C_{1-6}$-alkyl group, an $-SO-C_{1-6}$-alkyl group, an $-SO_2-C_{1-6}$-alkyl group, a $-C_{1-6}$-alkylene-$S-C_{1-6}$-alkyl group, a $-C_{1-6}$-alkylene-$SO-C_{1-6}$-alkyl group, a $-C_{1-6}$-alkylene-$SO_2-C_{1-6}$-alkyl group, an $-NH_2$-moiety, an NHR'-moiety or an NR'R''-moiety, wherein R' and R'' independently represent a linear or branched $C_{1-6}$-alkyl group, a $C_{1-6}$-alkyl group substituted by one or more hydroxy groups and a $-C_{1-6}$-alkylene-$NR^ER^F$ group,

whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,

$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a -CO-O- $C_{1-6}$-alkyl group, a - CO-NH- $C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a -CO-$NH_2$ group and/or which may contain at least one further heteroatom selected from the group consisting of nitrogen, oxygen and sulphur as a ring member, and

wherein $R^E$, $R^F$. Identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues $R^3$-$R^8$ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl. Br, I), a linear or branched $C_{1-6}$-alkyl group, a linear or branched $C_{1-6}$ alkoxy group, a formyl group, a hydroxy group, a trifluoromethyl group, a trifluoromethoxy group, a -CO-$C_{1-6}$-alkyl group, a cyano group, a carboxy group, a -CO-O-$C_{1-6}$-alkyl group, a -CO-$NR^AR^B$- moiety, a -CO-NH-$NR^CR^D$-moiety, an -S-$C_{1-6}$-alkyl group, an -SO-$C_{1-6}$-alkyl group, an -$SO_2C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, a $C_{1-6}$-alkyl group substituted by one or more hydroxy groups and a -$C_{1-6}$-alkylene-$NR^ER^F$, group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,

$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a -CO-O- $C_{1-6}$-alkyl group, a - CO-NH- $C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a -CO-$NH_2$ group and/or which may contain at least one further heteroatom selected from the group consisting of nitrogen, oxygen and sulphur as a ring member, and
wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo [2,1-b]thiazolyl, triazolyl and pyrazolyl.

If one or more of the residues $R^4$-$R^8$ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-4}$-alkoxy, branched or unbranched $C_{1-4}$-perfluoroalkoxy, branched or unbranched $C_{1-4}$-perfluoroalkyl, amino, carboxy, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methoxy, ethoxy, $CF_3$ and a phenyl group.

Preferred linear or branched, saturated or unsaturated aliphatic groups, which may be substituted by one or

more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues $R^4$-$R^8$ represents or comprises a linear or branched alkylene group, said alkylene group may preferably be selected from the group consisting of - methylene $-(CH_2)-$, ethylene $-(CH_2-CH_2)-$, n-propylene $-(CH_2-CH_2-CH_2)-$ or isopropylene $-(-C(CH_3)_2)-$.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}$ C-enriched carbon or $^{15}N$-enriched nitrogen are within the scope of this invention.

Most particularly preferred is the compound (R)-N-piperidinyl-5-(4-chloro-phenyl)-1-(2,4-dichlorophenyl)-4,5-dihydro-1H-pyrazol-3-carboxamide [compound 1]:

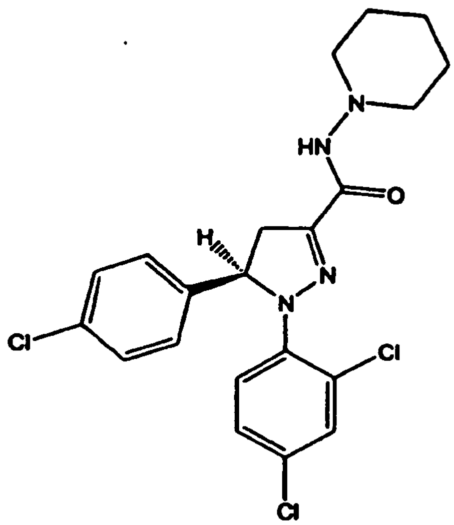

optionally in the form of a corresponding N-oxide, a corresponding salt or a corresponding solvate.

In another aspect the present invention also provides a process for the preparation of substituted pyrazoline compounds of general formula I given above, according to which at least one compound of general formula IIa

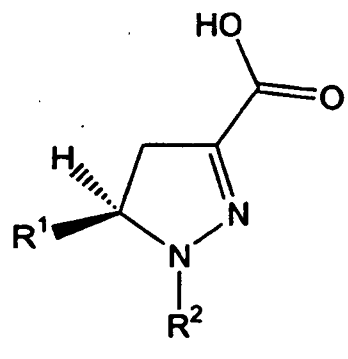

**(IIa)**

wherein $R^1$ and $R^2$ have the meaning given above, is optionally transferred under inert atmosphere to a compound of general formula (III) via reaction with an activating agent

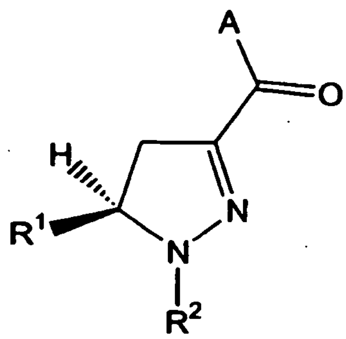

(III)

wherein the substituents $R^1$ and $R^2$ have the meaning given above and A represents a leaving group, preferably a chlorine atom, said compound being optionally isolated and/or optionally purified, and at least one compound of general formula (IIa) is reacted with a compound of general formula $R^3H$, wherein $R^3$ represents an $-NR^4R^5-$ moiety, with $R^4$ and $R^5$ having the meaning given above, under inert atmosphere to yield a substituted pyrazoline compound of general formula I, wherein $R^3$ represents an $-NR^4R^5$-moiety,
or at least one compound of general formula (III) is reacted with a compound of the general formula $R^3H$, in which $R^3$ has the meaning given above under inert atmosphere to yield a compound of general formula (I), which is optionally isolated and/or optionally purified.

Preferably the compound IIa is obtained from a mixture comprising the enantiomers

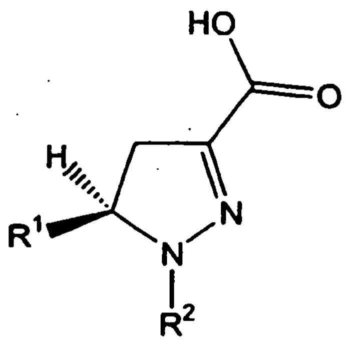

(IIa)

and

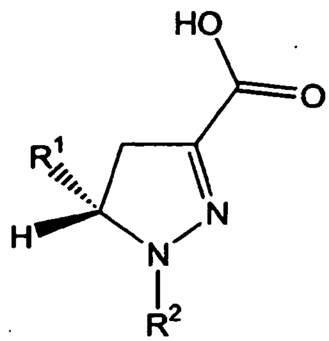

(IIb).

The compound (IIa) may be obtained from the mixture of the enantiomers by means well known to those skilled in the art. Preferably, the compound IIa is obtained from the mixture in form of an addition compound with a chiral base, preferably (+)-Cinchonine or R-(+)-1-Phenylethylamine, and preferably liberated from the addition compound.

Preferably the mixture comprising the enantiomers IIa and IIb may be obtained by reacting at least one benzaldehyde compound of general formula (IV)

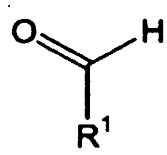

(IV)

wherein $R^1$ has the meaning given above, is reacted with a pyruvate compound of general formula (V)

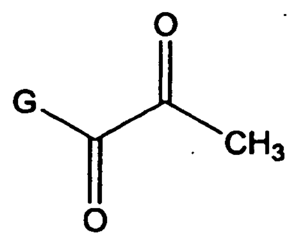

(V),

wherein G represents an OR group with R being a branched or unbranched $C_{1-6}$ alkyl radical, preferably an ethyl radical, or G represents an OK group with K being a cation, preferably a monovalent cation, more preferably an alkali metal cation, even more preferably a sodium cation, to yield a compound of general formula (VI)

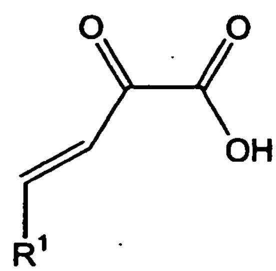

(VI)

wherein R' has the meaning given above, which is optionally isolated and/or optionally purified, and which is reacted with an optionally substituted phenyl hydrazine of general formula (VII)

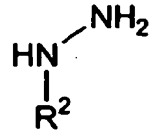

(VII)

or a corresponding salt thereof, wherein $R^2$ has the meaning given above, under inert atmosphere, to yield a mixture of compounds

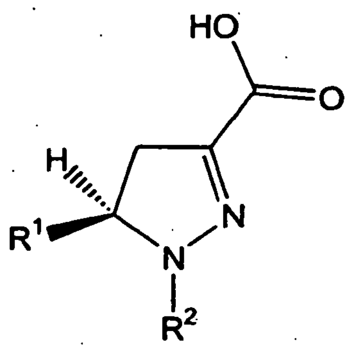

and

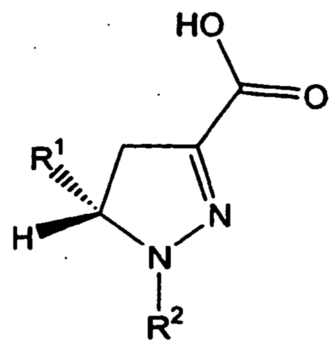

(IIa) (IIb).

The inventive process is also illustrated in scheme I given below:

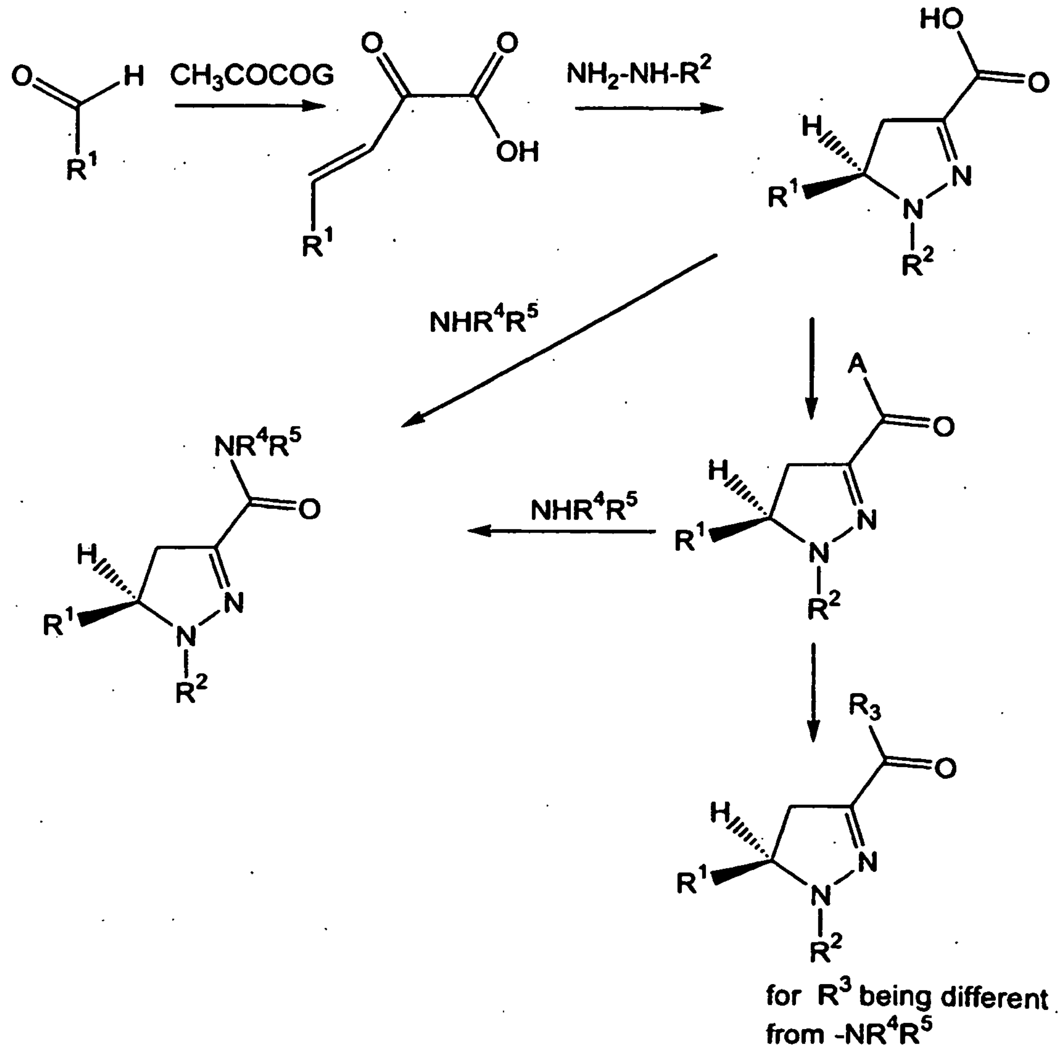


The reaction of the benzaldehyde compound of general formula IV with a pyruvate compound of general formula V is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula IV with a pyruvate compound of general formula V is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (VI) with an optionally substituted phenyl hydrazin of general formula (VII) is preferably carried out in a suitable reaction medium such as $C_{1-4}$-alcohols or ethers such as dioxane or tetrahydrofuran or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 ˚C to the boiling point of the reaction medium. Suitable reaction times may vary for example from several minutes to several hours.

The carboxylic group of the compound of general formula (III) may be activated for further reactions by the introduction of a suitable leaving group according to conventional methods well known to those skilled in the art. Preferably the compounds of general formula (VI) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (III) is an acid chloride, it is preferably prepared by reaction of the corresponding acid of general formula (IIa) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofuran or dimethoxyethane. Mixtures of two or more solvents from one class or two or more solvents from different classes may also be used. Preferred reaction temperature range from 0˚ C to the boiling point of the solvent and reaction times from several minutes to several hours.

If said activated compound of general formula (III) is a mixed anhydride, said anhydride may preferably be prepared, for example, by reaction of the corresponding acid of general formula (IIa) with ethyl chloroformiate in the presence of a base such as triethylamine or pyridine, in a suitable solvent.

The reaction of general formula (IIa) with a compound of general formula $HR^3$ to yield compounds of general formula I, wherein $R^3$ represents an $-NR^4R^5$ moiety is preferably carried out in presence of a base such as triethylamine in a reaction medium such as methylenchloride. The temperature is preferably in the range from 0˚C to the boiling point of the reaction medium. The reaction time may vary over a broad range, e.g. from several hours to several days.

The reaction of general formula (IIa) with a compound of general formula $HR^3$ to yield compounds of general formula I, wherein $R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341(7), 695-700; Lin, S. et al., Heterocycles; 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofuran or similar. The temperature is preferably in the range from 0˚C to the boiling point of the reaction medium, more preferably from 15 to 25 ˚C. The redaction time may vary over a broad range, e.g. from several minutes to several hours.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

In a further aspect the present invention also provides a process for the preparation of salts of substituted pyrazoline compounds of general formula (I), wherein at least one compound of general formula (I) having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula (I), wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes

which are complexed via ionic interactions. Especially this covers any physiologically acceptable salt.

Solvates, preferably hydrates, of the substituted pyrazoline compounds of general formula (I), of corresponding N-oxides or of corresponding salts thereof may also be obtained by standard procedures known to those skilled in the art.

Substituted pyrazoline compounds of general formula I, which comprise nitrogen-atom containing saturated, unsaturated or aromatic rings may also be obtained in the form of their N-oxides by methods well known to those skilled in the art.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

Those skilled in the art understand that substituents of the inventive compounds, particularly substituent $R^3$, may lead to stereoisomers, which are also covered by the present invention.

The purification and isolation of the inventive substituted pyrazoline compounds of general formula (I) and its intermediates, or a salt thereof, or an N-oxide thereof, or a solvate or any intermediate thereof may, if required, be carried out by conventional methods known to those skilled in the art, e.g. chromatographic methods or recrystallization.

The substituted pyrazoline compounds of general formula (I) given below, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula I given below, N-oxides thereof, corresponding salts and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these pyrazoline compounds show relatively weak Herg channel affinity, thus a low risk of prolongation of the QT-interval is to be expected for these compounds.

In summary, the inventively used pyrazoline compounds are distinguished by a broad spectrum of beneficial effects, while at the same time showing relatively little undesired effects, i.e. effects which do not positively contribute to or even interfere with the well being of the patient.

In particular, the chronic administration of the inventive CB1 antagonist (R)-N-piperidinyl-5-(4-chlorophenyl)-1-(2,4-dichlorophenyl)-4,5-dihydro-1-H-pyrazole-3-carboxamide significantly decreased body weight in animals made obese by exposure to a simplified cafeteria diet containing high-fat diet, chocolate and ground peanuts to mirror a typical Western diet. After 28 days of drug treatment, the body weight of rats given (R)-N-piperidinyl-5-(4-chlorophenyl)-1-(2,4-dichlorophenyl)-4,5-dihydro-1H-pyrazole-3-carboxamide was lower than those of the vehicle-treated controls.

Thus, an other aspect of the present invention relates to a medicament comprising at least one substituted pyrazoline compound of general formula I, optionally in form of a corresponding N-oxide thereof, or a corresponding salt thereof, or a corresponding solvate thereof, and optionally one or more pharmaceutically acceptable excipients. It is preferred that the inventive compounds are present in the medicament with an enantiomeric excess with respect to their other enantiomer (resulting from the stereocenter at the 5-position) of at least 90 %, more preferably at least 95 %, yet more preferably of at least 99 %. Most preferably, the inventive medicament comprises the substituted pyrazoline compound of general formula I in pure form, i.e. essentially free from its other enantiomer.

The inventive medicament may preferably also comprise any of the inventive pyrazoline compounds or combinations of at least two of these pyrazoline compounds given above.

Said medicament may also comprise any combination of one or more of the substituted pyrazoline compounds of general formula I given above, corresponding N-oxides thereof, physiologically acceptable salts thereof or physiologically acceptable solvates thereof.

Preferably said medicament is suitable for the modulation (regulation) of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferably said medicament is suitable for the prophylaxis and/or treatment of psychosis.

Also particularly preferably said medicament is suitable for the prophylaxis and/or treatment of food intake disorders, preferably bulimia, anorexia, cachexia, obesity and/or type II diabetus mellitus (non-insuline dependent diabetes mellitus), more preferably obesity. The inventive medicament also seems to be active in the prophylaxis and/or treatment of appetency disorders, e.g. the pyrazoline compounds of general formula I also reduce the desire for sweets.

Also particularly preferably said medicament is suitable for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said medicament is suitable for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Thus the inventive medicament is active in the treatment of abstinence, craving reduction and relapse prevention of alcohol intake. The inventive medicament can also be used in the prophylaxis and/or treatment of smoking addiction, cessation and/or dependence including treatment for craving reduction and relapse prevention of tobacco smoking.

Medicaments and/or drugs, which are frequently the subject of misuse include opioids, barbiturates, cannabis, cocaine, amphetamines, phencyclidine, hallucinogens and benzodiazepines.

The medicament is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhoea, asthma, memory disorders, pruritus, pain, or for potentiation of the analgesic effect of narcotic and non-narcotic analgesics, or for influencing intestinal transit.

The medicament is also suitable for the prophylaxis and/or treatment of one or more disorders selected from the group consisting of dementia and rotated disorders, preferably for the prophylaxis and/or treatment of one or more types of dementia selected from the group consisting of memory loss, vascular dementia, mild cognitive impairment, frontotemporal dementia and Pick's disease; binge eating disorders; juvenile obesity; drug induced obesity; atypical depression; behavioural addictions; attention deficit disorders; Tourette's syndrome; suppression of reward-related behaviours; e. g. conditioned place avoidance such as suppression of cocaine- and morphine induced conditioned place preference; impulsivity; sexual dysfunction; preferably for the prophylaxis and/or treatment of one or more types of sexual dysfunction selected from the group consisting of erectile difficulty and female sexual dysfunction; seizure disorders; nausea; emesis; neuroinflammatory disease, preferably for the prophylaxis and/or treatment of one or more types of neuroinflammatory diseases selected from the group consisting of multiple sclerosis, demyelinisation related disorders, Guillan-Barré syndrome, viral encephalitis and cerebrovascular accidents; neurological disorders; muscle spasticity; traumatic brain injury; spinal cord injury; inflammation and immunomodulatory disorders, preferably for the treatment and/or prophylaxis of one or more types of inflammation and immunomodulatory disorders selected from the group consisting of cutaneous T-cell lymphoma, rheumatoid arthritis, systemic lupus erythematosus, sepsis, sarcoidosis, idiopathic pulmonary fibrosis, bronchopulmonary dysplasia, retinal disease, scleroderma, renal ischemia, mycocardial infarction, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, Crohn's disease, inflammatory bowel disease, reversible airway obstruction, adult respiratory distress syndrome, chronic obstructive pulmonary disease and bronchitis; cerebral apoplexy; craniocerebral trauma; neuropathic pain disorders; gastric ulcers; atheriosclerosis and liver cirrhosis.

Another aspect of the present invention is the use of at least one substituted pyrazoline compound of general formula I given above as suitable active substances, optionally in form of a corresponding N-oxide thereof, or a corresponding salt thereof, or a corresponding solvate thereof, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of psychosis.

Also particularly preferred is the use of at least one of the respective pyrazoline compounds, optionally in form

of a corresponding N-oxide thereof, or a corresponding salt thereof, or a corresponding solvate thereof, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also particularly preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also particularly preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of at least one of the respective pyrazoline compounds, optionally in form of a corresponding N-oxide thereof, or a corresponding salt thereof, or a corresponding solvate thereof, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, , medicament-induced movement disorders, dystonia, endotoxemic shock, hemorrhagic shock, hypotension, insomnia, immunologic disorders, sclerotic plaques, vomiting, diarrhoea, asthma, memory disorders, pruritus, pain, or for potentiation of the analgesic effect of narcotic and non-narcotic analgesics, or for influencing intestinal transit.

Also particularly preferred is the use of at least one of the of the respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the preparation of a medicament for the prophylaxis and/or treatment of one or more disorders selected from the group consisting of dementia and related disorders, preferably for the prophylaxis and/or treatment of one or more types of dementia selected from the group consisting of memory loss, vascular dementia, mild cognitive impairment, frontotemporal dementia and Pick's disease; binge eating disorders; juvenile obesity; drug induced obesity; atypical depression; behavioural addictions; attention deficit disorders; Tourette's syndrome; suppression of reward-related behaviours; e. g. conditioned place avoidance such as suppression of cocaine- and morphine induced conditioned place preference; impulsivity; sexual dysfunction; preferably for the prophylaxis and/or treatment of one or more types of sexual dysfunction selected from the group consisting of erectile difficulty and female sexual dysfunction; seizure disorders; nausea; emesis; neuroinflammatory disease, preferably for the prophylaxis and/or treatment of one or more types of neuroinflammatory diseases selected from the group consisting of multiple sclerosis, demyelinisation related disorders, Guillan-Barré syndrome, viral encephalitis and cerebrovascular accidents; neurological disorders; muscle spasticity; traumatic brain injury; spinal cord injury; inflammation and immunomodulatory disorders, preferably for the treatment and/or prophylaxis of one or more types of inflammation and immunomodulatory disorders selected from the group consisting of cutaneous T-cell lymphoma, rheumatoid arthritis, systemic lupus erythematosus, sepsis, sarcoidosis, idiopathic pulmonary fibrosis, bronchopulmonary dysplasia, retinal disease, scleroderma, renal ischemia, mycocardial infarction, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, Crohn's disease, inflammatory bowel disease, reversible airway obstruction, adult respiratory distress syndrome, chronic obstructive pulmonary disease and bronchitis; cerebral apoplexy; craniocerebral trauma; neuropathic pain disorders; gastric ulcers; atheriosclerosis and liver cirrhosis.

Dementia is a disease characterized by the progressive deterioration in cognitive and social adaptive functions that can eventually interfere with the patient's ability to live independently. Dementia also constitutes of impairment in short- and long-term memory plus additional symptoms, such as problems with abstract thinking, judgment, or personality.

An estimated 18 million patients suffer from dementia worldwide. The most common forms of dementia include Alzheimers disease and vascular dementia. Other forms are frontotemporal dementia and Pick's disease.

Dementia can also be of vascular origin. Vascular dementia (atherosclerotic cerebrovascular disease) is considered to be the second most common dementia of late life, affecting approximately 10-15% of all cases. AD and vascular dementia can exist in isolation or together (mixed dementia). In vascular dementia, atherosclerotic changes in cerebral vessels can lead to reduced local blood flow that results in multiple small strokes (multi-infarct dementia). Vascular dementia is pharmacologically treated by stroke prophylaxis, and by treatment of the cognitive deficit.

Alzheimer's disease (AD), the most common and important form of dementia, is a neurodegenerative disorder that is characterized by progressive impairment of cognitive functions, such as abstract reasoning and memory. Currently, an estimated 2 million people in the United States and 12 million worldwide are afflicted by this disease. Due to increasing life expectancy, it is predicted that there will be over 100 million AD patients worldwide by the year 2050. AD is one of the most prevalent illnesses in the elderly. The majority of AD patients are in their sixties or older. More than 5% of all persons over the age of 70 have significant memory loss due to AD.

AD is mainly characterized through a gradual development of forgetfulness. In further advanced disease stages, other failures in cerebral function become increasingly apparent. This includes impairment of speech, writing, and arithmetic skills. Visiospacial orientation, such as parking the car, dressing properly, and giving and understanding directions to a location, can become defective or impaired. In late stage disease, patients forget how to use common objects and tools while retaining necessary motor power and co-ordination for these activities.

Schizophrenia is characterized by profound disruption in cognition and emotion, affecting the most fundamental human attributes: language, thought, perception, affect, and sense of self. Positive symptoms include psychotic manifestations, such as hearing internal voices or experiencing other sensations not connected to an obvious source (hallucinations) and assigning unusual significance or meaning to normal events or holding fixed false personal beliefs (delusions). Negative symptoms are characterized by affective flattening and lack of initiative or goals (avolition), loss of usual interests or pleasures (anhedonia), disturbances of sleep and eating, dysphoric mood (depressed, anxious, irritable, or angry mood) and difficulty concentrating or focusing attention.

Major depression is a multifaceted disorder characterized by primarily by dysphoric mood and loss of interest or pleasure in activities that were once enjoyable. Other physical and psychological symptoms include inability to concentrate, motor disturbances (psychomotor retardation or agitation), feelings of worthlessness, inappropriate guilt, thoughts of suicide, and disturbances in appetite and sleep.

Anxiety disorders are a group of syndromes that include generalized anxiety disorder, panic disorder, phobias, obsessive-compulsive disorder, and post traumatic stress disorder. Although each disorder has its own distinct features, all share common symptoms of excessive worrying, intense fears and dread, hypervigilance and/or somatic symptoms, in the absence of a dangerous situation.

Normal sexual function requires, among others, the ability to achieve and maintain penile erection. Major anatomic structures of the penis that are involved in erectile function include the corpus cavemosum, corpus spinosum, and the tunica albuginea (a collagenous sheath that surrounds each corpus). Thecorpora are composed of a mass of smooth muscle (trabecula) which contains a network of endothelial-lined vessels (lacunar spaces). Penile tumescence and erection is caused by relaxation of the arteries and corporal smooth muscles, while closing emissary veins, leading to increased blood flow into the lacunar network. Central and peripheral innervation contributes to regulation of the erectile response.

Erectile dysfunction (ED) may result from failure to initiate, fill, or store adequate blood volume'within the lacunar network of the penis. Depending on the underlying dysfunction, ED may be vasculogenic, neurogenic, endocrinologic, diabetic, psychogenic, or medication-related.

ED affects 10-25% of middle-aged and elderly men, and has a profound impact on the well-being of affected men. It is currently treated using PDE5 inhibitors such as vardenafil, tadalifil, and sildenafil. Intraurethral alpostadil (prostaglandin EI) may be used in patients that fail on oral agents. In addition, vacuum constriction devices (VCD) are a well-established, noninvasive therapy.

Female sexual dysfunction (FSD) is highly prevalent, age-related, and progressive. It affects 30 to 50% of women. FSD denotes a range of medical problems and is categorized according to disorders of (1) desire, (2) arousal, (3) orgasm and (4) sexual pain, and symptoms include diminished vaginal lubrication, pain and discomfort with intercourse, decreased arousal, and difficulty achieveing orgasm. On a molecular level, vasoactive intestinal peptide (VIP), nitic oxide (NO), and sex hormones such as estrogens and androgens have been suggested to be important in female sexual function. Current treatment approaches include estrogen replacement therapy, methyl testosterone, PDE5 inhibitors such as sildenafil, the NO-donor L-arginine, prostaglandin EI, phentolamine, and the dopamine agonists apomorphine.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilising agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

**Pharmacological Methods:**

**I. In-vitro determination of affinity to CB1/CB2-Receptors**

a)

The in-vitro determination of the affinity of the inventive substituted pyrazoline compounds to $CB_1$/$CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

b)

**Rat cerebellum CB1 binding**

Binding affinity to CB1 receptor was evaluated according to a modification of the method described by Govaerts et al., Eur J Pharmac Sci 23, 233-243 (2004). The respective parts of the description is hereby incorporated by reference and forms part of the present disclosure.

Briefly, cerebellum from male wistar rats (250-300g) were carefully dissected on ice and homogenates were prepared with Potter-Helveheim in a cold 50 mM Tris-HCl solution containing 5 mM $MgCl_2$, 1 mM EDTA and 0.25 M sucrose, pH 7.4. The suspension was centrifuged at 1,000 x g for 5 minutes. The supernatants were collected and centrifuged 50,000 x g for 15 minutes. The resulting pellets were then resuspended in Tris-HCl buffer without sucrose, homogenized and incubated for 15 min at 37°C in an orbital shaker bath and centrifuged again at 50,000 x g for 15 min. Pellets were weighted, resuspended in Tris-HCl buffer without sucrose, homogenized with Ultraturrax at 13,500 rpm for 3 x 5 seconds and alicuoted in 0.9 ml volumes in Eppendorf tubes. Alicuotes were centrifuged at 20,800 x g for 5 minutes, supernatants discarded and pellets were frozen at -80°C until use. Total protein concentration was determined using the Bio-Rad Lowry method based kit. Competitive binding experiments were performed in presence of 1 nM [$^3$H]-CP 55,940 in siliconized glass tubes containing 100 μg protein/tube resuspended in 1ml final volume of 50 mM Tris-HCl, 5 mM $MgCl_2$ , 1 mM EDTA, 0.5% (w/v) bovine serum albumin, pH 7.4. Compounds were present at various concentrations and the non specific binding was determined in the presence of 10 μM HU-210. After 1 hour incubation at 30°C, the suspension was rapidly filtered through 0.5% PEI pre-treated GF/B fiber filters on a 96-well harvester and washed 3 times with 3 ml ice-cold binding buffer without bovine serum albumin. Radioactivity on filters was measured with Wallac Winspectral 1414 counter by liquid scintillation in 6 ml Ecoscint H (National Diagnostics, U.K.). Assays were made in triplicates.

Binding data were analyzed by non-linear regression with the software GraphPad Prism Version 3.03.

## II. In-vivo bioassay system for determination of cannabinoid activity

### Mouse tetrad model

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are highly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

### Material and Methods

Male NMRI mice with a weight of 20-30 g (Harlan, Barcelona, Spain) are used in all of the following experiments.

Before testing in the behavioural procedures given below, mice are acclimatised to the experimental setting. Pre-treatment control values are determined for analgesia hot plate latency (in seconds), rectal temperature, sedation and catalepsy.

In order to determine the agonistic activity of the substance to be tested, the mice are injected intravenously with the substance to be tested or the vehicle atone. 15 minutes after injection, latency in hot plate analgesia is measured.

Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injected 5 minutes before the intravenous injection of 1.25 mg/kg Win-55,212 a known cannabinoid-receptor agonist.

### Hot plate analgesia

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 $\pm$ 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minutes after the administration of the substance to be tested, the mice are again placed on the hot plate and the afore described procedure is repeated. This period is called the post-treatment reading (PT).

The degree of analgesia is calculated from the formula :

$$\text{\% MPE of Analgesia} = (PT - B) / (PC - B) \times 100$$

MPE = Maximum possible effect.

### Determination of sedation and ataxia

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.

The chosen scoring system is

0: no ataxia;
1: doubtful;
2: obvious calmness and quiet;
3 pronounced ataxia;

prior to as well as after treatment.

**The percentage of sedation is determined according to the formula:**

$$\% \text{ of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277, 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥ -2 ˚C are considered to represent activity.

**Catalepsy:**

Catalepsy is determined according to the method described in Alpermann H. G. et al. "Pharmacological effects of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:

more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according to the following formula:

$$\% \text{ Catalepsy} = \text{arithmetic mean} / 6 \times 100$$

**III. In vivo testing for antiobesic activity**

a) Accute Treatment

Normally handled rats were habituated to a reversed cycle 12/12h, and the tested compound as well as saline was acutely orally administered. After administration the cumulated food intake (g) was measured at 6 h and 24 h. Following that the difference in body weight between control and compound treated animals was measured. This is a variation of the test according to Colombo et al. as described below.

b) Long-term Treatment

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

## IV. In vivo testing for antidepressant activity

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

## V. Determination of the effect of continued administration of the inventive compounds on body weight in rats

The weight of the animals was measured for 36 days, and the data grouped into 3 periods for analysis: Period 1, days 1-8 (period for adaptation of the animals to the environmental conditions: one week without treatment plus first day of treatment); Period 2, days 9-22 (treatment period: two weeks); Period 3, days 23-36 (escape treatment period: two weeks). The administration of the drugs started on day 8, immediately after the animal was weighted, and finished on day 21. The treatment period ranges between one day after first administration and one day after last administration. The drugs were administered i.p. once daily. For comparative purposes, absolute weights were transformed into relative percentages (each weight was divided by that of day 8 and multiplied by 100). Three treatment groups were constructed: vehicle, racemic N-piperidinyl-5-(4-chlorophenyl)-1-(2,4-dichlorophenyl)-4,5-dihydro-1H-pyrazole-3-carboxamide (10 mg/kg), and (R)-N-piperidinyl-5-(4-chlorophenyl)-1-(2,4-dichlorophenyl)-4,5-dihydro-1H-pyrazole-3-carboxamide (10 mg/kg). The number of animals was 10 for the (R)-N-piperidinyl-5-(4-chlorophenyl)-1-(2,4-dichlorophenyl)-4,5-dihydro-1H-pyrazole-3-carboxamide group and 9 for the racemic N-piperidinyl-5-(4-chlorophenyl)-1-(2,4-dichlorophenyl)-4,5-dihydro-1H-pyrazole-3-carboxamide group and for the vehicle group. In the vehicle group one animal died whereas in the racemic group one animal was excluded because its weight decreased during 5 days in the post-treatment period. All results are expressed as mean ± S.E.M.

The dependence of body-weight (expressed as a percentage relative to day 8) on time (number of days after inclusion in the study) and treatment was assessed by a two-way (time, treatment) analysis of variance (ANOVA) with repeated measures on the time factor. The analysis was performed on periods 2 and 3, separately, and considering the first or second week in each period. The differences between pairs of curves (treatments) were analyzed using appropriate contrasts within the ANOVA test. The possible interaction between time and treatment was also considered including the Greenhouse-Geisser (G-G) correction. A value of $P < 0.05$ was considered significant.

**Figure 1** shows the effect of continued administration (i.p. once daily) of racemic N-piperidinyl-5-(4-chlorophenyl)-1-(2,4-dichlorophenyl)-4,5-dihydro-1H-pyrazole-3-carboxamide (A), (R)-N-piperidinyl-5-(4-chlorophenyl)-1-(2,4-dichlorophenyl)-4,5-dihydro-1H-pyrazole-3-carboxamide (B) and vehicle on body weight in rats. The R-compound shows a stronger action than the racemate as well as a slower recovery of weight.

## VI. In vitro determination of antagonism to CB1-receptor

### Membrane preparation:

Chinese hamster ovary (CHO) cells stably expressing recombinant human cannabinoid 1 receptor (CB1) were cultured in nutrient mixture Ham's F 12 supplemented with 10 % heat-inactivated fetal bovine serum, 2 mM L-glutamine, 50 U/ml penicillin, 50 U/ml streptomycin and 0.5 mg/ml geneticin. In order to obtain cells, culture flasks were washed twice with phosphate buffered saline and scraped. Then, cells were collected by centrifugation (200 x g, 10 min) and stored dry at -80°C. Cells were homogenized in ice-cold 20 mM HEPES, 10 mM EDTA (pH 7.5) and centrifuged at 40,000 x g for 15 min at 4°C. The membrane pellet was resuspended in 20 mM HEPES, 0.1 mM EDTA (pH 7.5) and centrifuged for 15 min at 4°C. The final membrane pellet was resuspended in 20 mM HEPES, 0.1 mM EDTA (pH 7.5), and divided in aliquots and stored at -80°C until use.

### [$^{35}$S]GTPγS binding assay:

The reaction was performed in 96-well plates. Membranes (15 μg protein/well) were incubated for 60 min at 30 °C in buffer (50 mM HEPES, 100 mM KCl, 5 mM $MgCl_2$,1 mM EDTA, 0.1 % wt/vol bovine serum albumin, 5 μM GDP, saponin (10 μg/ml), 0.5 nM [$^{35}$S]GTPγS, pH 7.4) with compound at a final concentration of 1 μM in either the absence or presence of agonist WIN 55,212-2 between 3 nM and 3 μM. The incubation was terminated by rapid filtration through Millipore Multiscreen glass fiber FB, and rinsed two-times with ice-cold assay buffer. Filter plates were dried and 30 μl of scintillation liquid was added. Radioactivity was determined using a Wallac Microbeta Trilux. Each experiment was performed at least in duplicate. A WIN 55,212-2 dose-response experiment either alone or in the presence of Rimonabant (1 μM) was systematically performed.

**Calculations:**

The average of basal [$^{35}$S]GTPγS binding was subtracted from all binding data. In order to compare the antagonism results from one screening campaign to another one, the difference between the maximal agonist effect of WIN 55,212-2 alone, and the maximal antagonism effect due to WIN 55,212-2 plus Rimonabant (1 μM) was defined as 100 %.

**Further methods:**

**Alcohol Intake**

The following protocol may be used to evaluate the effects of alcohol intake in alcohol preferring (P) female rats (e.g. bred at Indiana University) with an extensive drinking history. The following reference provides detailed a description of P rats: Lumeng, L, et al.,"Different sensitivities to ethanol in alcohol-preferring and-nonpreferring rats,"Pharmacol, Biochem Behav., 16, 125-130 (1982).

Female rats are given 2 hours of access to alcohol (10% v/v and water, 2-bottle choice) daily at the onset of the dark cycle. The rats are maintained on a reverse cycle to facilitate experimenter interactions. The animals are initially assigned to four groups equated for alcohol intakes: Group 1-vehicle; Group 2-positive control (e. g. 5.6 mg/kg AM251; Group3-low dose test compound; and Group 4-high dose of test compound. Test compounds are generally mixed into a vehicle of 30% (w/v) - cyclodextrin in distilled water at a volume of 1-2 ml/kg. Vehicle injections are given to all groups for the first two days of the experiment. This is followed by 2 days of drug injections (to the appropriate groups) and a final day of vehicle injections. On the drug injection days, drugs are given sc 30 minutes prior to a 2-hour alcohol access period. Alcohol intake for all animals is measured during the test period and a comparison is made between drug and vehicle-treated animals to determine effects of the compounds on alcohol drinking behavior.

Additional drinking studies can be done utilizing female C57Bl/6 mice (Charles River). Several studies have shown that this strain of mice will readily consume alcohol with little to no manipulation required (Middaugh et al., "Ethanol Consumption by C57BU6 Mice : Influence of Gender and Procedural Variables" Alcohol, 17 (3),175-183, 1999; Le et al., "Alcohol Consumption by C57BL/6, BALA/c, and DBA/2 Mice in a Limited AccessParadigm" PharmacologyBiochemistry and Behavior,47, 375-378,1994).

For example, upon arrival mice are individually housed and given unlimited access to powdered rat chow, water and a 10 % (w/v) alcohol solution. After 2-3 weeks of unlimited access, water is restricted for 20 hours and alcohol is restricted to only 2 hours access daily. This is done in a manner that the access period was the last 2 hours of the dark part of the light cycle.

Once drinking behavior is stabilized, testing can commence. Mice are considered stable when the average alcohol consumption for 3 days is 20% of the average for all 3 days. Day 1 of test consists of all mice receiving vehicle injection (sc or ip). Thirty to 120 minutes post injection access is given to alcohol and water. Alcohol consumption for that day is calculated (g/kg) and groups are assigned so that all groups have equivocal alcohol intake. On day 2 and 3, mice are injected with vehicle or drug and the same protocol as the previous day is followed. Day 4 iss wash out and no injections are given. Data is analyzed using repeated measures ANOVA. Change in water or alcohol consumption is compared back to vehicle for each day of the test. Positive results would be interpreted as a compound that was able to significantly reduce alcohol consumption while having no effect on water

**Oxygen Consumption Methods:**

Whole body oxygen consumption is measured using an indirect calorimeter (Oxymax from Columbus Instruments, Columbus, OH) in male Sprague Dawley rats (if another rat strain or female rats are used, it will be specified). Rats (e.g. 300-380 g body weight) are placed in the calorimeter chambers and the chambers are placed in activity monitors. These studies are done during the light cycle. Prior to the measurement of oxygen consumption, the rats are fed standard chow ad libitum. During the measurement of oxygen consumption, food is not available. Basal pre-dose oxygen consumption and ambulatory activity are measured every 10 minutes for 2.5 to 3 hours. At the end of the basal pre-dosing period, the chambers are opened and the animals are administered a single dose of compound (the usual dose range is 0.001 to 10 mg/kg) by oral gavage (or other route of administration as specified, i. e. , sc, ip, iv). Drugs are prepared in methylcellulose, water or other specified vehicle (examples include PEG400, 30% beta-cyclo dextran and propylene glycol). Oxygen consumption and ambulatory activity are measured every 10 minutes for an additional 1-6 hours post-dosing.

The Oxymax calorimeter software calculates the oxygen consumption (ml/kg/h) based on the flow rate of air through the chambers and difference in oxygen content at inlet and output ports. The activity monitors have 15 infrared light beams spaced one inch apart on each axis, ambulatory activity is recorded when two consecutive beams are broken

and the results are recorded as counts.

Resting oxygen consumption, during pre-and post-dosing, is calculated by averaging the 10-min02 consumption values, excluding periods of high ambulatory activity (ambulatory activity count > 100) and excluding the first 5 values of the pre-dose period and the first value from the post-dose period. Change in oxygen consumption is reported as percent and is calculated by dividing the post-dosing resting oxygen consumption by the pre-dose oxygen consumption *100. Experiments will typically be done with n = 4-6 rats and results reported are mean +/-SEM.

Interpretation :

An increase in oxygen consumption of > 10% is considered a positive result. Historically, vehicle-treated rats have no change in oxygen consumption from pre-dose basal.

**Nicotine Dependence**

An intravenous nicotine self-administration model or place preference model may be used to assess the effects of a test compound on nicotine dependence (see, e.g., Vastola, et al. Physiol. Behav. 77:107-114, 2002; Brower, et al., Brain Res. 930:12-20, 2002).

**Place Preference**

Sprague-Dawley rats are used in this study (Vastola, et al., 2002). Animals are housed in a temperature-controlled, 12h/12h illumination cycle with ad libitum access to food and water. Conditioning and testing are conducted in a chamber divided into two compartments with a door separating the two compartments. Behavior of the animals is recorded by video camera.

Animals are habituated to the injection procedure for several days. The animals are then placed into the test chamber and given free access to both compartments. The initial preference for a particular compartment is determined. For the conditioning trials, animals are injected with nicotine and restricted to the nonpreferred compartment, or the animals are injected with saline and restricted to the preferred compartment. On test day, the door separating the compartments is removed, the animal is placed in the center of the chamber and allowed to move freely between compartments. Time spent in each compartment is scored. Preferential occupancy of the nicotine compartment follows from the conditioned reinforcing effects of nicotine.

**Self-administration**

Self-administration in animals is a predictor of a compound's abuse potential in humans. Modifications to this procedure may also be used to identify compounds that prevent or block the reinforcing properties of drags that have abuse potential. A compound that extinguishes the self- administration of a drag may prevent that drag's abuse or its dependence.

Sprague-Dawley rats are used in this study. Initially, animals are housed in a temperature- controlled, 12h/12h illumination cycle with ad libitum access to food and water. The animals are then implanted with jugular catheters which exit through the animal's back, and each animal is placed in an individual operant chamber (Brower, et al., 2002). The catheters are connected to a computer-driven syringe pump which is located outside of the chamber. The chamber contains two levers with a green light located above each lever. The light is illuminated when nicotine is available.

In a self-administration test, animals are placed in the operant chambers and the levers are randomly designated as an active and inactive lever. Each response on the active lever produces an infusion of nicotine. Presses on the inactive lever have no effect, but are also recorded. Animals are then trained to self -administer nicotine over a set period of time by having drag access during each daily session. Illumination of the chamber house light signals the beginning of the session and the availability of nicotine. When the session ends, the house light is turned off. Initially, a nicotine infusion occurs with every press of the active lever. Once lever-pressing behavior has been established, the number of presses to produce a nicotine infusion is increased. After stable nicotine self-administration is obtained, the effect of a test compound on the nicotine- reinforced behavior may be evaluated. Administration of this test compound prior to the session can either potentiate, extinguish, or produce no change to the self-administrating behavior. Tests are conducted every two days, and the order of the administration of the test compound doses is controlled.

## ALZHEIMER/DEMENTIA EXPERIMENTS

### Morris Water Maze Task

The Morris water maze is a behavioral in vivo test to measure spatial orientation learning and memory through a complex learning task. It is highly suitable for testing compounds that enhance learning and memory. A circular water tank or pool (diameter 2 m, height 0.7 m) is filled with water, and a 10 cm2 platform is placed 1-1.5 cm below the water surface at a defined location within the pool. The escape platform is not visible for an animal swimming in the water tank. For the experiment, a rat or mouse is placed into the pool to swim freely.

The animals have the task to localize the submerged platform, and the time and distance required for successful retrieval is measured. Multiple extra-maze cues are provided by the furniture in the room, including desks, computer equipment, a second water tank, the presence of the experimenter, and by a radio on a shelf that is playing softly.

Before administration of the test compound, animals are usually trained in the task 4 times a day for 5 days. Test compounds are administered orally or intraperitoneally on the day of the experiment at a defined time (e.g., 30 minutes before the first swim test). Control animals are dosed with the corresponding vehicle not containing test compound. Active compounds yield shorter times and distances to localize the platform (i.e., the better the animal remembers the location of the platform, the shorter the distance covered and the faster the platform is reached).

The test can also be carried out using transgenic or cognitively impaired animals. Cognitive impairment is induced either by old age or experimentally through brain lesions, such as bilateral lesions of the entorhinal cortex in rats. Such lesions can be induced by intracerebral injections of the excitotoxin ibotenic acid.

### Object Recognition Task

The object recognition task is used to assess the effects of compounds on the cognitive performance of rodents. A rat is placed in an open field, in which two identical objects are located. The rats inspects both objects during the initial trial of the test. After a certain retention interval (e.g., 24 hours), a second trial is carried out. Here, one of the two objects used in the first trial (the 'familiar' object) and a novel object are placed in the open field, and the inspection time at each of the objects is measured. Good retention is reflected by higher exploration times towards the novel compared with the 'familiar' object.

Administration of the putative cognition enhancer prior to the first trial predominantly allows assessment of the effects on acquisition, and on the consolidation processes. Administration of the test compound after the first trial allows to assess the effects on consolidation processes, whereas administration before the second trial allows to measure effects on retrieval processes.

### Passive Avoidance Task

The passive avoidance task assesses memory performance in rats and mice. The inhibitory avoidance uses an apparatus consisting of a box with two compartments separated by a guillotine door that can be operated by the experimenter. One compartment is illuminated with bright light, and the other compartment is dark. A threshold of 2 cm separates the two compartments when the guillotine door is 15 raised. When the door is open, the illumination in the dark compartment is about 2 lux. The light intensity is about 500 lux at the center of the floor of the light compartment.

Two habituation sessions, one shock session, and a retention session are given, separated by inter-session intervals of 24 hours. During the habituation sessions and the retention session, the rat is allowed to explore the apparatus for 300 seconds. The rat is placed in the light compartment, facing the wall opposite to the guillotine door. After an accommodation period of 15 seconds, the guillotine door is opened so that all parts of the apparatus can be visited freely. Rats normally avoid brightly lit areas and will enter the dark compartment within a few seconds.

In the shock session, the guillotine door between the compartments is lowered as soon as the rat has entered the dark compartment with all paws, and a scrambled 1 mA footshock is administered for 2 seconds. Then the rat is removed from the apparatus and returned into its home cage. The procedure during the retention session is identical to that of the habituation sessions.

The step-through latency, that is, the first latency of entering the dark compartment (in seconds) during the retention session is an index of the memory performance of the animal: a better retention is assumed if the latency to enter the dark compartment is longer. A test compound is given 30 minutes before the shock session, together with 1 mg/kg scopolamine. Scopolamine impairs the memory performance during the retention session 24 hours later. If the test compound increases the enter latency compared with the scopolamine-treated controls, it is considered to possess cognition enhancing activity. T-maze Spontaneous Alternation Task

The T-maze spontaneous alternation task (TeMCAT) assesses the spatial memory performance in mice. The start arm and the two goal arms of the T-maze are provided with guillotine doors that can be operated manually by the

experimenter. A mouse is put into the start arm at the beginning of training. In the first trial, either the left or right goal arm is blocked by lowering the respective guillotine door (forced trial).

After the mouse has been released from the start arm, it will explore the maze, eventually entering the open goal arm, and return to the start position, where it will be confined for 5 seconds, by lowering the guillotine door. Then, the animal can choose freely between the left and right goal arm (all guillotine-doors opened) during 14 additional trials (free choice trials). As soon as a mouse has entered one goal arm, the other arm is closed. The mouse eventually returns to the start arm and is free to visit whichever arm it wants after having been confined to the start arm for 5 seconds. After completion of 14 free choice trials in one session, the animal is removed from the maze.

Out of the 14 trials the alternations in percent are calculated. This percentage and the total time needed to complete the first forced trial and the subsequent 14 free choice trials (in seconds) is analyzed. In addition, cognitive deficits can be induced by injection of scopolamine 30 minutes before the start of the training session. A cognition enhancer, administered before the training session, will at least partially, antagonize the scopolamine-induced reduction in the spontaneous alternation rate.

**Depression Model**

A forced swim or tail suspension model may be used to assess the efficacy of antidepressant compounds (see , e.g., Porsolt, et al., Nature 266:730-732, 1977; Stem, et al., Psychopharmacology 85:367-370, 1985).

**Forced Swim Test**

Rats or mice are placed in a cylinder filled with water 23-25 ˚C from which no escape is possible. Initially, animals struggle and try to escape, but eventually adopt a characteristic immobile posture and make no further attempts to escape except for small movements needed their head above water. Animals are dosed with a compound and the activity (swimming or climbing) or immobility is measured by an observer. The immobility is considered by some to reflect a 'behavioral despair in which animals cease to struggle to escape the aversive situation. A wide variety of clinically used antidepressants (TCAs, MAOIs, SSRIs, atypicals) decrease immobility in this test and has a good predictive validity in that it detects antidepressants with different mechanisms of action but its construct validity is weak. At least two distinct active behavioral patterns are produced by pharmacologically selective antidepressant drugs. Serotonin-selective reuptake inhibitors increase swimming behavior, whereas drugs acting primarily to increase extracellular levels of norepinephrine or dopamine increase climbing behavior. There are false positives (psychostimulants) but relatively few false negatives ([beta]-adrenergic agonists). The test is sensitive to muscle-relaxant (benzodiazepines) and sedative (neuroleptics) effects, leading to enhanced immobility. False positives and false negatives can often be screened by measuring if the compound produces locomotor stimulation or sedation.

**Tail Suspension Test**

When suspended by the tail, mice will initially struggle and try to escape and then alternate between active escape attempts and immobililty. In this test, animals are dosed with a compound and the immobility is measured by an observer for 6 min. Porsolt describes the immobile behavior as 'behavioral despair' which animals cease to struggle to escape the aversive situation. A large variety of clinically antidepressants (tricyclics, MAOIs, SSRIs, and atypicals) reduce immobility in this model. The test has a good predictive validity for antidepressant activity and works for most antidepressant classes including but has some false positives (psychostimulants). The test is sensitive to muscle-relaxant (benzodiazepines) and sedative (neuroleptics) effects, which lead to enhanced immobility. False positives and false negatives can often be screened by measuring if the compound produces locomotor stimulation or sedation. Strain differences in the tail suspension test have been found in mice. The tail suspension test has some face validity but its construct validity is rather weak.

**Schizophrenia Model**

A prepulse inhibition model may be used to assess the efficacy of antipsychotic compounds (see Swerdlow and Geyer, Schizophrenia Bulletin 24: 285-301, 1998).

**Prepulse Inhibition**

Prepulse inhibition is the process whereby a relatively mild stimulus, the prepulse, suppresses the response to a strong, startle-eliciting stimulus when the prepulse precedes the startle stimulus by a brief duration (about 10 to 500 milliseconds). Prepulse inhibition is a cross-species phenomenon (ie, it is present in mammals ranging from mice to

humans), yet it is relatively absent among schizophrenic patients. The deficit in PPI in schizophrenic patients is thought to reflect the loss of sensorimotor gating that may lead to sensory flooding and cognitive fragmentation. In this test, mice or rats are administered compounds and individually placed into a holder on a transducer platform to measure whole body startle. The holder is housed in a startle chamber with background white noise. Following a brief habituation period, animals are given multiple trials of a weak auditory prepulse stimululs, followed by a strong auditory startle stimulus. Four types of trials are given: prepulse plus startle, prepulse alone, startle alone, and no stimulation. PPI is measured as the amount of inhibition of startle following the prepulse and is expressed as the percentage of basic startle. As a control, measurements are taken in the no stimulation and prepulse alone trials. PPI is considered a test with good predictive, face and construct validity for schizophrenia. Putative antipsychotics can be tested alone to determine if they enhance PPI. Alternately, antipsychotics can be screened to determine if they block various agents that disrupt PPI (apomorphine, d-amphetamine, PCP, ketamine, DOI). Finally, mutant mice with or without drugs can be screened using the PPI procedure.

**Anxiety Model**

An elevated plus maze model may be used to assess the efficacy of anxiolytic compounds (see Pellow and File, Pharm. Biochem. Behav. 24, 525-529, 1986).

**Elevated Plus Maze**

The elevated plus maze is widely used as an anxiety paradigm that examines the conflict between the drive to explore and the aversiveness of heights and open spaces of rats or mice. The maze is a cross made up of two open and two closed arms that is raised above the ground. The combination of light, the open arms, and the height is thought to produce unconditioned fear or anxiety responses in mice or rats. The test apparatus is an open top maze constructed of opaque plastic with alternating open and enclosed arms. For rats, each arm is 45-55 cm long and 8-12 cm wide, with the sides of the enclosed arms 35-45 cm high, the juncture approximately 10 x 10 cm, and the maze is elevated 45-55 cm above the floor. The mouse elevated plus maze consists of two closed arms (15 x 6 x 30 cm) and two open arms (1 x 6 x 30 cm) forming a cross, with a quadrangular center (6 x 6cm). The maze is placed 50 cm above the floor. Testing is performed in a room free of noise and distraction. On test days animals are administered drug or vehicle. If a pretreatment period is necessary, the animals are returned to the home cage for the duration of the pretreatment time; otherwise, the animals are placed in a clear plastic holding chamber singly or with cage mates for 1-10 minutes prior to test time. Rats are then placed in the center of the maze always oriented in the same direction, either consistently facing an open arm or an enclosed arm. Four 5-10 minutes, entries into each arm and the time spent in each arm are recorded by the observer(s) or by videotape or a computer receiving input from a video camera mounted above the maze. To count as an entry, all four paws must be inside the arm. If necessary, additional measures of anxiety-related behaviors will be recorded, i.e., time spent motionless, time spent in the center, time spent grooming, and the number of rears, stretching postures or feces produced. Following testing the animals are returned to the home cages. When animals are placed in the center of the maze, they spend most of their time in the closed arms, avoiding the open arms. Anxiolytic drugs, such as benzodiazepines, will increase the amount of time animals spend in the open arms. The test is also sensitive to anxiogenic drugs, which lends strong support for its predictive validity.

**Erectile Dysfunction**

Drugs affecting erectile function may be tested by measuring the effect on apomorphine- evoked increases in intracavemous pressure in the awake rat as described by Andersson, et al., (J. Urol. 161 : 1707-17] 2, 1999). One end of a polyethylene tubing is implanted into the cavemosal space of the penis of male Sprague-Dawley rats. After recovery from the surgery, intracavemous pressure is recorded using a pressure transducer connected to a multichannel pen-recorder. Erections are induced by administration of apomorphine (100-250 ug/kg s.c.) with or without test compound, and the results are compared for the treated group and the non-treated group.

**Female Sexual Dysfunction**

Systems to test compounds for the treatment of female sexual dysfunction include in vitro and in situ models using vaginal or clitoral smooth muscle preparations, histological evaluation, and vaginal blood flow assessments. In vivo studies of sexual responses focus on behavioral paradigms involving lordotic posturing and receptivity, as well as indices of motivation using a dual chamber pacing method (see, e.g., Hale, et al., Int. J. Impot. Res. 15 Suppl 5: S75-79, 2003).

The present invention is illustrated below with the aid of examples. These illustrations are given solely by way

of example and do not limit the general spirit of the present invention.

**Examples:**

**Abbreviations:**

| | |
|---|---|
| Eq. | Equivalent |
| Proc. | Process |
| Solv. Cryst. | Solvent used in crystallisation experiment |
| T Cryst. | Temperature at which crystallisation experiment was carried out |
| $1^{st}$ Cryst. | first crystallisatioN |
| $2^{nd}$ Crys. | second crystallisation |
| r.t. | room temperature (≈ 20-25 °C) |

**Example 1:**

**General synthesis of racemic N-piperidinyl-5-(4-chlorophenyl)-1-(2,4-dichlorophenyl)-4,5-dihydro-1H-pyrazole-3-carboxamide**

**a) 4-(4-chlorophenyl)-2-oxo-3-butenoic acid**

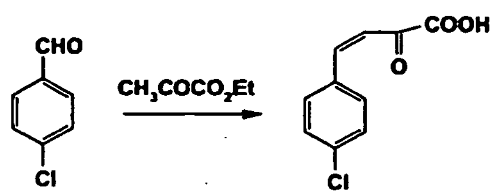


In a three neck flask p-chlorobenzaldehyde (13,3 g, 95 mmoles) and ethyl pyruvate (10 g, 86 mmoles) were dissolved in 150 ml of absolute ethanol. The solution was ice-cooled to 0°C and an aqueous solution of NaOH (3.8 g in 45 mL water) was added dropwise keeping the temperature below or equal to 10°C, whereby a yellow-orange coloured precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).
IR (KBr, $cm^{-1}$) : 3500-2500, 1719,3, 1686,5, 1603,4, 1587,8, 1081,9.
$^1$H NMR($CDCl_3$, δ) : 7,4 (d, J=8,4Hz, 2H), 7,5 (d, J=16,1Hz, 1H), 7,6 (d, J=8,4Hz, 2H), -8,1(d, J=16,, 1H).

**b) 5-(4-chlorophenyl)-1-(2,4-dichlorophenyl)-4,5-dihydro-pyrazole-3-carboxylic acid**

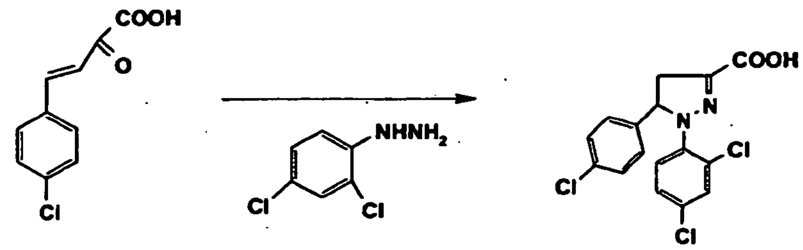

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, $cm^{-1}$) : 3200-2200, 1668,4, 1458, 1251,4, 1104,8.

$^1H$ NMR ($CDCl_3$, $\delta$) : 3,3 (dd, 1H), 3,7 (dd, 1H), 5,9 (dd, 1H), 7,09-7,25 (m, 7H).

**(c) 5-(4-chlorophenyl)-1-(2,4-dichlorophenyl)-4,5-dihydro-pyrazole-3-carboxylic acid chloride**

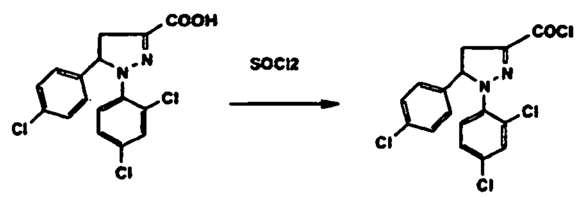


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, $cm^{-1}$) : 1732,3, 1700, 1533,3, 1478,1, 1212,9, 826,6.

**d) N-piperidinyl-5-(4-chlorophenyl)-1-(2,4-dichlorophenyl)-4,5-dihydropyrazole-3-carboxamide** [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

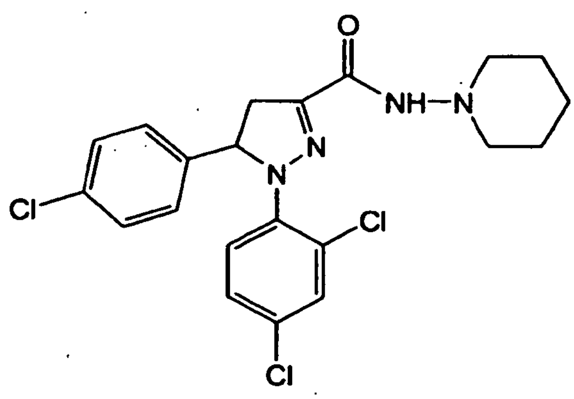

Under nitrogen atmosphere N-aminopiperidine (0.6 mL, 5.6 mmoles) and triethylamine (4 mL) were dissolved in methylene chloride (25 mL). The resulting mixture was ice-cooled down to 0˚C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 ˚C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186˚C.
IR (KBr, $cm^{-1}$) : 3222,9, 2934,9, 1647,4, 1474,7, 1268,3, 815,6.
$^1H$ NMR ($CDCl_3$, δ) : 1,4 (m, 2H), 1,7 (m, 4H), 2,8 (m, 4H), 3,3 (dd, J=6,1 y 18,3Hz, 1H), 3,7 (dd, J=12,5 and 18,3 Hz, 1H), 5,7 (dd, J=6,1 and 12,5 Hz, 1H), 7,0-7,2 (m, 6H), 7,4 (s, 1H).

The resolution of the racemate of N-piperidinyl-5-(4-chlorophenyl)-1-(2,4-dichlorophenyl)-4,5-dihydro-1H-pyrazole-3-carboxamide may be carried out by methods known to those skilled in the art, e.g. column chromatography.

However, is has been found by the present inventors that its intermediate, namely racemic 5-(4-chlorophenyl)-1-(2,4-dichlorophenyl)-4,5-dihydro-pyrazole-3-carboxylic acid may easily be separated into the respective enantiomers via reaction with a chiral base. The process for this resolution is described below

**Example 2:**

**Resolution of the enantiomers of 5-(4-chlorophenyl)-1-(2,4-dichlorophenyl)-4,5-dihydro-pyrazole-3-carboxylic acid**

The resolution of the enantiomers of 5-(4-chlorophenyl)-1-(2,4-dichlorophenyl)-4,5-dihydro-pyrazole-3-carboxylic acid was carried out via reaction with the following chiral bases:

- Brucine
- Quinine
- (-)-Cinchonidine
- (+)-Cinchonine
- R-(+)-1-Phenylethylamine
- (1 R,2S)-(-)-Ephedrine hydrochloride
- (1S,2R)-(+)-Ephedrine hydrochloride.

In each case the reactions were carried out with 0.5 and 1 equivalents of base in respect to 1 equivalent of the acid compound and by using the following solvents

- Ethanol
- Acetone
- Acetonitril
- Dioxane
- Ethylacetate
- Chloroform.

The results are summarized in the following tables. It may be understood that the afore mentioned crystallisation experiments that are not reflected in the following tables did not yield crystals of the respective salts under the given conditions.

However, suitable conditions for crystallization of these salts can be determined by those skilled in the art via routine experiments.

In the following tables
Acid represents racemic 5-(4-chlorophenyl)-1-(2,4-dichlorophenyl)-4,5-dihydro-pyrazole-3-carboxylic acid
R-Acid represents the respective derivative of (R)-5-(4-chlorophenyl)-1-(2,4-dichlorophenyl)-4,5-dihydro-pyrazole-3-carboxylic acid
S-Acid represents the respective derivative of (S)-5-(4-chlorophenyl)-1-(2,4-dichlorophenyl)-4,5-dihydro-pyrazole-3-carboxylic acid

**Processes for crystallisation:**

**Process A:** A solution of the chiral base was added on top of a solution of the racemic acid at room temperature.

**Process C:** A solution of the racemic acid was added on top of a solution of the chiral base. The mixture was heated to reflux and solvent was added until dissolution was complete. The solution was left to crystallisation at r.t.

**Process D:** The chiral base was directly added on top of a solution of the racemic acid at room temperature.

**Process E:** The chiral base was directly added on top of a solution of the racemic acid at reflux temperature.

**Process F:** The solution of the salt was evaporated to dryness. The residue was dissolved in a minimum amount of the solvent under reflux heating. The solution was left to crystallisation at r.t.

**Resolution with Brucine**

Brucine

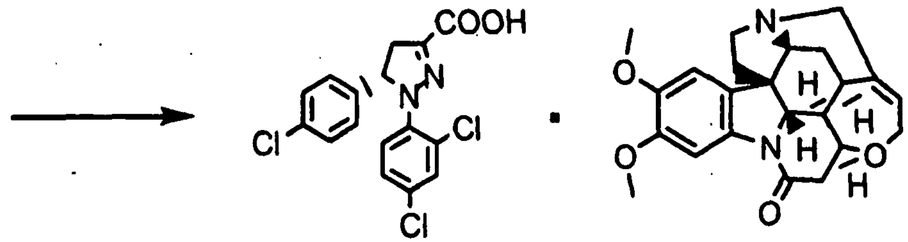

| Acid g (mmol) | Eq. amine | Proc. | Solvent for crystallisation | T Cryst. | Yield 1st Cryst. % | % S-Acid | % R-Acid | Yield 2nd Cryst. % | % S-Acid |
|---|---|---|---|---|---|---|---|---|---|
| 0,4 g (1,09 mmol) | 0,5 | A | 8 ml acetone | r.t. | 44,6 | 98,8 | 1,2 | 5,6% | 99,4 |
| 0,4 g (1,09 mmol) | 0,5 | A | 11,5 ml acetonitrile | r.t. | 50,7 | 47,5 | 52,4 | | |
| 0,4 g (1,09 mmol) | 1 | A | 17 ml acetonitrile | r.t. | 25,6 | 45,7 | 54,3 | | |

**Resolution with Quinine**

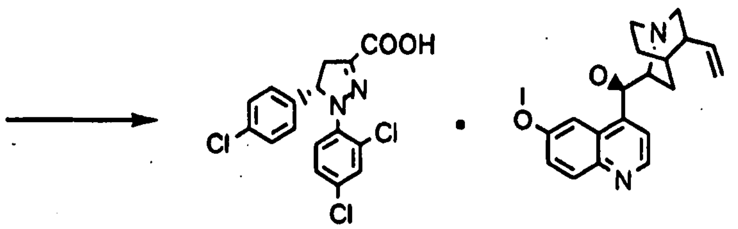

| Acid g (mmol) | Eq. amine | Proc. | Solvent for crystallisation | T Crys. | Yield 1st Cryst. % | % S-Acid | % R-Acid |
|---|---|---|---|---|---|---|---|
| 0,4 g (1,09 mmol) | 1 | A | 8 ml dioxane | r.t. | 49,46 | 91,6 | 8,3 |
| 0,4 g (1,09 mmol) | 1 | F | 15ml acetonitrile | r.t. | 26 | 94,4 | 5,5 |
| 0,4 g (1,09 mmol) | 1 | F | 2ml ethylacetate | r.t. | 25 | 96,7 | 3,3 |
| 0,4 g (1,09 mmol) | 0,5 | A | 4ml dioxane | r.t. | 38,5 | 97,5 | 2,5 |

**Resolution with (-)-Cinchonidine**

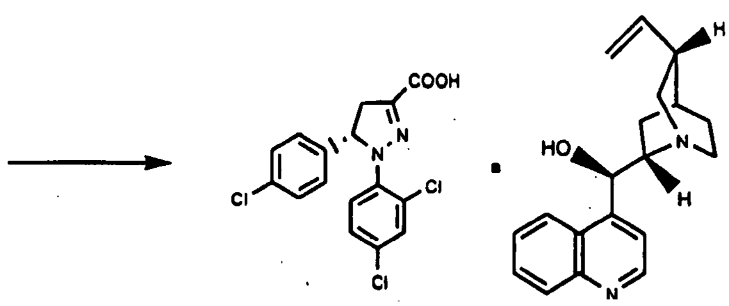

| Acid g (mmol) | Eq. amine | Proc. | Solvent for crystallisation | T Cryst. | Yield 1st Cryst. % | % S-Acid | % R-Acid |
|---|---|---|---|---|---|---|---|
| 0,4 g (1,09 mmol) | 1 | F | 2ml dioxane | r.t. | 31 | 94,4 | 5,6 |
| 0,4 g (1,09 mmol) | 1 | F | 19ml Ethylacetate | r.t. | 28,5 | 95,8 | 4,2 |
| 0,4 g (1,09 mmol) | 1 | F | 20ml acetone | r.t. | 19,6 | 96,9 | 3,1 |
| 0,4 g (1,09 mmol) | 1 | F | 24ml acetonitrile | r.t. | 42 | 85,8 | 14,1 |

**Resolution with (+)-Cinchonine**

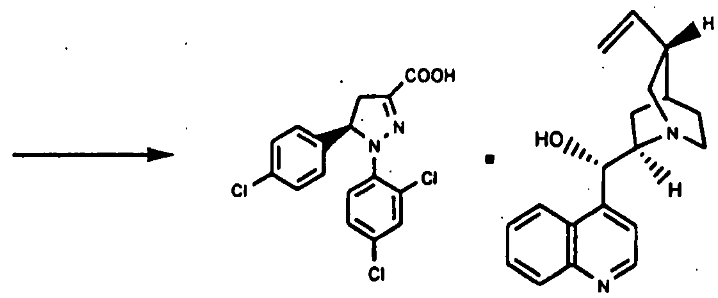

| Acid | Eq. amine | Proc. | Solvent for crystallisation | T Cryst | Yield 1st Cryst. % | % S-Acid | % R-Acid |
|---|---|---|---|---|---|---|---|
| 0,4 (1,09 mmol) | 1 | C | 50 ml acetone | r.t. | 32,8 | 7,42 | 92,57 |
| 0,4 (1,09 mmol) | 0,5 | F | 30ml acetone | r.t. | 23,5 | 4,0 | 95,9 |
| 0,4 (1,09 mmol) | 0,5 | C | 50 ml acetonitrile | r.t. | 31,5 | 3,07 | 96,92 |
| 0,4 (1,09 mmol) | 1 | C | 50 ml acetonitrile | r.t. | 78,25 | 39,01 | 60,98 |
| 0,4 (1,09 mmol) | 0,5 | C | 15 ml ethylacetate | r.t. | 14,9 | 3,62 | 96,37 |
| 0,4 (1,09 mmol) | 1 | C | 27 ml ethylacetate | r.t. | 35 | 7,78 | 92,21 |
| 0,4 (1,09 mmol) | 1 | F | 4ml dioxane | r.t. | 21 | 33,5 | 66,4 |

**Resolution with R-(+)-1-Phenylethylamine**

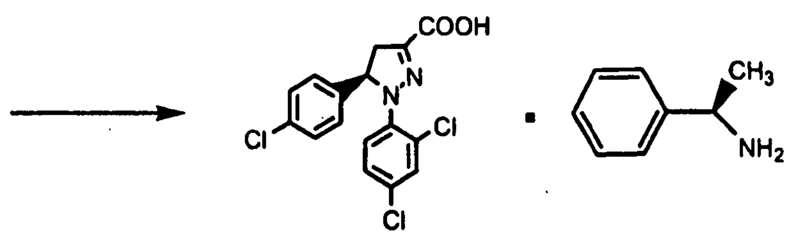

| Acid | Eq. amine | Proc. | Solvent for crystallisation | T Cryst. | Yield 1st Cryst. % | % S-Acid | % R-Acid |
|---|---|---|---|---|---|---|---|
| 0,4 (1,09 mmol) | 1 | D | 1,6ml ethanol | r.t. | 22 | 51,4 | 48,6 |
| 0,4 (1,09 mmol) | 0,5 | E | 6ml acetonitrile | r.t. | 11 | 8,1 | 91,9 |
| 0,4 (1,09 mmol) | 1 | E | 6ml acetonitrile | r.t. | 50 | 36,8 | 63,2 |

(continued)

| Acid | Eq. amine | Proc. | Solvent for crystallisation | T Cryst. | Yield 1st Cryst. % | % S-Acid | % R-Acid |
|---|---|---|---|---|---|---|---|
| 0,4 (1,09 mmol) | 1 | E | 6ml dioxane | ≈5˚C | 5 | 4,6 | 95,3 |

**Detailed description:**

**Resolution of 5-(4-chlorophenyl)-1-(2,4-dichlorophenyl)-4,5-dihydro-pyrazole-3-carboxylic acid with (+)-Cinchonine**

35 g (294.68 mmol) of racemic 5-(4-chlorophenyl)-1-(2,4-dichlorophenyl)-4,5-dihydro-pyrazole-3-carboxylic acid in 630 m acetonitril were added to a suspension of 16.39 g (47.34 mmol) of (+)-Cinchonine in 920 ml acetonitrile under vigorous stirring. The resulting suspension was heated to reflux and acetonitrile (1300 ml) was added until dissolution was complete. The resulting solution was left to crystallise at room temperature over the weekend, whereupon a crystalline solid was obtained. The crystalline solid was filtered off, washed with 50 ml of cold acetonitrile and dried to give 25.26 g of a white solid (ee ≈ 91-92%). No second fraction of crystals could be obtained from the mother liquors, neither upon cooling in the refrigerator nor upon concentration. Thus, recrystallization of the diastereomeric salt was carried out in different solvents in order to improve the enantiomeric excess:

| Solvent | Yield | Ratio of enantiomers % (R) / % (S) |
|---|---|---|
| Acetonitrile | 90 % | 99,6 / 0,4 |
| EtOH-$H_2O$ | 68.5 % | 99,3 / 0,7 |
| Isopropanol | 55 % | 98,9 / 1,1 |

Consequently, the product was recrystallized from acetonitrile to give 22.6 g of crystals (yield 72 mol% related to chiral base). The ratio of enantiomers determined by capillary electrophoresis was:

99.7 % of (R)-5-(4-chlorophenyl)-1-(2,4-dichlorophenyl)-4,5-dihydro-pyrazole-3-carboxylic acid (+)-Cinchonine
0.3 % of (S)-5-(4-chlorophenyl)-1-(2,4-dichlorophenyl)-4,5-dihydro-pyrazole-3-carboxylic acid (+)-Cinchonine ee= 99.4 %.

**Preparation of (R)-5-(4-chlorophenyl)-1-(2,4-dichlorophenyl)-4,5-dihydro-pyrazole-3-carboxylic acid from addition compound (R)-5-(4-chlorophenyl)-1-(2,4-dichlorophenyl)-4,5-dihydro-pyrazole-3-carboxylic acid-(+)-Cinchonine**

22.59 g (34 mmol) of the diastereomeric salt, which was recrystallized from acetonitrile, were suspended in 200 ml of 6N HCl and stirred at room temperature for 15 minutes. Afterwards, 200 ml of toluene were added until dissolution was complete. The mixture was stirred for 30 minutes and the phases were separated. The aqueous phase was extracted with toluene, the combined organic phases were washed with water, dried over sodium sulfate, filtered and evaporated to dryness, whereupon 11.9 g (95 %) of a white microcrystalline solid (melting point 129-133) were obtained.
Enantiomeric excess determined bY capillary electrophoresis. ee = 99.2 % Chemical purity determined by HPLC: 99,3 %
$[\alpha]_D$ (c=1,23˚C, MeOH) = -429.

**Isolation of the enantiomer (R)-5-(4-chlorophenyl)-1-(2,4-dichlorophenyl)-4,5-dihydro-pyrazole-3-carboxylic acid from the mother liquors obtained from the crystallisation of the enantiomer (S)-5-(4-chlorophenyl)-1-(2,4-dichlorophenyl)-4,5-dihydro-pyrazole-3-carboxylic acid with Brucine**

The residue obtained from the evaporation of the mother liquors obtained from the crystallisation of (S)-5-(4-chlorophenyl)-1-(2,4-dichlorophenyl)-4,5-dihydro-pyrazole-3-carboxylic acid with Brucine was treated with 6N HCl as described above for obtaining the free acid. After evaporating the organic phase 21.86 g of a mixture with a theoretical composition of 20 mmoles (S)-5-(4-chlorophenyl)-1-(2,4-dichlorophenyl)-4,5-dihydro-pyrazole-3-carboxylic acid and

39.9 mmoles of (R)-5-(4-chlorophenyl)-1-(2,4-dichlorophenyl)-4,5-dihydro-pyrazole-3-carboxylic acid were obtained. A suspension of 13.82 g (39.9 mmoles) of (+)-cinchonine in 1000 ml of acetonitrile were added and the mixture was heated to reflux. More acetonitrile was added until dissolution was complete (total volume of acetonitrile 4000 ml). The mixture was then slowly cooled to room temperature to obtain crystals. After a few hours a white solid was obtained, which was filtered off and dried to give 22.42 (yield 84,5% related to the salt of (R)-5-(4-chlorophenyl)-1-(2,4-dichlorophenyl)-4,5-dihydro-pyrazole-3-carboxylic acid). ,

Enantiomeric excess as determined by capillary electrophoresis was ee = 92 %.

The purity of the isolated salt was comparable to the one of the salt obtained directly from the resolution of the racemate. Consequently, the same purification process as described above could be applied.

**Resolution of racemic 5-(4-chlorophenyl)-1-(2,4-dichlorophenyl)-4,5-dihydro-pyrazole-3-carboxylic acid with Brucine**

**Preparation of the diastereomeric salt with Brucine**

At room temperature 15.74 g (39.9 mmol) of Brucine were dissolved in 475 ml of acetone under vigorous stirring. A solution of 29.5 g (79.8 mmol) of the racemic 5-(4-chlorophenyl)-1-(2,4-dichlorophenyl)-4,5-dihydro-pyrazole-3-carboxylic acid in 85 ml acetone was added. The resulting mixture was allowed to crystallise for a couple of days, whereupon a crystalline solid was obtained. The crystalline solid was filtered off, washed with cold acetone and dried to yield 13.66 g (45 mol % related to the base) of a beige-coloured solid were obtained.

The ratio of the enantiomers as determined by capilar electrophoresis was:

99 % of (S)-5-(4-chlorophenyl)-1-(2,4-dichlorophenyl)-4,5-dihydro-pyrazole-3-carboxylic acid Brucine
1 % of (R)-5-(4-chlorophenyl)-1-(2,4-dichlorophenyl)-4,5-dihydro-pyrazole-3-carboxylic acid Brucine
Enantiomeric excess ee = 98 %

The filtered mother liquors were left in the refrigerator and yielded a second fraction of crystals, which were filtered off and dried to give another 1.56 g (5.1 molar % related to the base).

The analysis of the $2^{nd}$ fraction via capillar electrophoresis gave
99.3 % of (S)-5-(4-chlorophenyl)-1-(2,4-dichlorophenyl)-4,5-dihydro-pyrazole-3-carboxylic acid Brucine
0.7 % of (R)-5-(4-chlorophenyl)-1-(2,4-dichlorophenyl)-4,5-dihydro-pyrazole-3-carboxylic acid Brucine
Enantiomeric excess ee = 98.6 %

Total yield:

15.22 g (50.1 mol-% related to base) of the salt with ee = 98 %.

**Determination of the chirality of the Brucine salt via X-ray crystallography**

The chirality of the enantiomer that crystallized with Brucine (ee 98 %) was determined via x-ray crystallography and corresponds to the S-enantiomer. Accordingly, the other enantiomer that crystallized with (+)-cinchonine is the corresponding R-enantiomer.

**Preparation of (S)-5-(4-chlorophenyl)-1-(2,4-dichlorophenyl)-4,5-dihydro-pyrazole-3-carboxylic acid from addition compound (S)-5-(4-chlorophenyl)-1-(2,4-dichlorophenyl)-4,5-dihydro-pyrazole-3-carboxylic acid brucine**

15.17 g (19.85 mmol) of (S)-5-(4-chlorophenyl)-1-(2,4-dichlorophenyl)-4,5-dihydro-pyrazole-3-carboxylic acid Brucine were suspended in 150 ml of 6N HCl and the suspension was stirred for 10 minutes, followed by the addition of 150 ml of toluene upon which complete dissolution was observed. The resulting mixture was stirred for an additional 30 minutes under control with column chromatography (silical gel, $Cl_2CH_2$:MeOH 95:5). Afterwards, the phases were separated, the aqueous phase was extracted with toluene and the combined toluene phases were washed with water three times, dried over sodium sulfate, filtered and evaporated to dryness. 7 g (95,5 %) of an oil were obtained, which solidified upon addition of a small amount of diethylether to yield a white amorphous solid that showed a crystalline transformation under melting at 130-133 ˚C.
CCF($Cl_2CH_2$:MeOH 95:5) : $R_f$ = 0.3

The analysis of the enantiomers via capillar electrophoresis gave
99.1 % (S)-5-(4-chlorophenyl)-1-(2,4-dichlorophenyl)-4,5-dihydro-pyrazole-3-carboxylic acid

0.9 % (R)-5-(4-chlorophenyl)-1-(2,4-dichlorophenyl)-4,5-dihydro-pyrazole-3-carboxylic acid
Enantiomeric excess ee = 98.2 %
$[\alpha]_D$ (c=1,23˚C, MeOH) = -480.5.
Chemical purity determined by HPLC: 99.1 %
$^1$H-NMR ($CDCl_3$) δ ppm: 3,2 (dd, J=6,4 y 18,2 Hz, 1H), 3,7 (dd, J=12,7 y 18,2 Hz, 1H), 5,85 (dd, J=6,4 y 12,6 Hz, 1H), 7,0-7,2 (m, 7H).

**Isolation of the enantiomer (S)-5-(4-chlorophenyl)-1-(2,4-dichlorophenyl)-4,5-dihydro-pyrazole-3-carboxylic acid from the mother liquors obtained from the crystallisation of the other enantiomer (R)-5-(4-chlorophenyl)-1-(2,4-dichlorophenyl)-4,5-dihydro-pyrazole-3-carboxylic acid with (+)-cinchonine**

The residue obtained from the evaporation of the mother liquors obtained from the crystallisation of (R)-5-(4-chlorophenyl)-1-(2,4-dichlorophenyl)-4,5-dihydro-pyrazole-3-carboxylic acid with (+)-Cinchonine was treated with 6N HCl as described above for obtaining the free acid. After evaporating the organic phase 23.63 g of a mixture with a theoretical composition of 13.34 mmoles of (R)-5-(4-chlorophenyl)-1-(2,4-dichlorophenyl)-4,5-dihydro-pyrazole-3-carboxylic acid and 47.34 mmoles of (S)-5-(4-chlorophenyl)-1-(2,4-dichlorophenyl)-4,5-dihydro-pyrazole-3-carboxylic acid were obtained. Said mixture, dissolved in 118 ml of acetone, was added on top of a solution of 18.7 g (47.4 mmoles) of brucine in 650 ml of acetone, stirred for a couple of minutes and left to crystallise at room temperature overnight. A creme-coloured solid was obtained that was filtered off and dried to yield 28 g (77.5 % relative to the (S)-salt).

Enantiomeric excess as determined by capilar electrophoresis was ee = 98.2 %

The purity of the isolated salt was comparable to the one of the salt obtained directly from the resolution of the racemate. Consequently, the same purification process as described above could be applied.

**Preparation of (R)-N-piperidinyl-5-(4-chlorophenyl)-1-(2,4-dichlorophenyl)-4,5-dihydro-1H-pyrazole-3-carboxamide (A)**

9.94 g (26.89 mmol) of (R)-5-(4-chlorophenyl)-1-(2,4-dichlorophenyl)-4,5-dihydro-pyrazole-3-carboxylic acid were dissolved in 95 ml of toluene, followed by the addition of 2.35 ml (32.3 mmoles) of thionyl chloride and 4 drops of DMF. Under anhydrous nitrogen atmosphere the mixture was heated to a temperature of 75-80˚ C under vigorous stirring for two hours and then allowed to cool to room temperature. Said acid chloride solution was then added dropwise to a solution of 3.47 ml (31.2 mmol) of N-Aminopiperidine, 15 ml (107.6 mmol) of triethylamine and 38 ml of anhydrous toluene, which was cooled to 0-5 ˚C, thereby keeping the temperature below 10 ˚C during the addition. The reaction mixture was stirred at room temperature overnight, whereupon a suspension was obtained. Water and Toluene were added to said suspension until dissolution was complete and the phases were separated. The aqueous phase was extracted with toluene, the combined organic phases washed with an NaOH solution (10 %) and water, dried over sodium sulfate, filtered off and evaporated to dryness to yield 13.41 g of light yellow oil, which failed to crystallise. The oil was purified via column chromatography over silica gel (eluent: petrol ether ethylacetate 90: 10 to 60: 40) to give 10.5 g (83.5 %) of (R)-N-piperidinyl-5-(4-chlorophenyl)-1-(2,4-dichlorophenyl)-4,5-dihydro-1H-pyrazole-3-carboxamide in form of an amorphous solid of light yellow colour (melting point 75-78 ˚C).
$^1$H-NMR (DMSO-$d_6$) δ ppm : 9,25 (s, 1H), 7,5 (d, J=8,8Hz, 1H), 7,4 (d, J=2,3Hz, 1H), 7,3-7,25 (2d, J=8,8 and 8,5Hz, 3H), 7,1 (d, J=8,5Hz, 2H), 5,8 (dd, J=5,7 and 11,8 Hz, 1H), 3,65 (dd, J=11,8 and 18,1Hz, 1H), 3,0 (dd, J=5,7 and 18,1Hz, 1H), 2,75 (m, 4H), 1,5 (m, 4H), 1,2 (m, 2H).
Analysis of the enantiomer via chiral HPLC: ee =100 %
Chemical purity determined by HPLC: 98,5%
$[\alpha]_D$ (c=1, 23˚C, MeOH) = - 293,5

**Preparation of Hydrochloride salt of (R)-N-piperidinyl-5-(4-chlorophenyl)-1-(2,4-dichlorophenyl-4,5-dihydro-1H-pyrazole-3-carboxamide (A-Hydrochloride)**

0.5 g (1.1 mmol) of (R)-N-piperidinyl-5-(4-chlorophenyl)-1-(2,4-dichlorophenyl)-4,5-dihydro-1H-pyrazole-3-carboxamide were dissolved in 10 ml isopropanol, cooled with ice andistsaturated HCl-ethanol solution was added. The solution changed its colour and a white solid formed, which was filtered off, washed with isopropanol:diethylether (1:1) and dried to yield 0.38 g (71 %) of a white solid with a melting point of 160-165 ˚C. The purity determined by HPLC was 99.3 %. Determination of chlorine: 7.15 % (98.5 % of the theoretical value).
$^1$H-NMR (DMSO-$d_6$) δ ppm : 10,3 (bs, 1H), 7,5 (m, 2H), 7,3 (2d, J= 2,5 y 8,5Hz, 3H), 7,15 (d, J=8,5Hz, 2H), 5,8 (dd, J=6,3 y 12,0Hz, 1H), 3,7 (dd+$H_2O$), 3,05 (m, 5H), 1,7 (m, 4H), 1,4 (m, 2H).

**Pharmacological Data**

The binding of pyrazoline compounds of general formula I to the CB1-receptor was determined as described in the section Pharmacological methods, part I.

The pyrazoline compounds of general formula I show a high affinity to the CB1-receptor (table 1).

**Table 1**.

| **Compound according to example** | **$IC_{50}$** [nM] | **Inhibition [%], $10^{-7}$** M | **Inhibition [%], $10^{-8}$** M |
|---|---|---|---|
| A | 16.1 | | |
| A-Hydrochloride | 20 | | |

The antagonism of the pyrazoline compounds of general formula I to the CB1-receptor was determined according to the method described in Pharmacological methods, part VI (table 2).

Table 2.

| **Compound according to example** | **Antagonism [%]** |
|---|---|
| A | 106 |
| A-Hydrochloride | 113 |

**Claims**

1. Compounds of general formula I

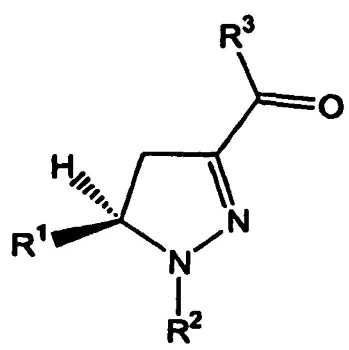

I

wherein

$R^1$ represents a phenyl group, which is optionally substituted by one or more substituents independently selected from the group consisting of methyl, ethyl, F, Cl, Br and $CF_3$,
$R^2$ represents a phenyl group, which is optionally substituted by one or more substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more $C_{1-6}$-alkyl groups, or $R^3$ represents an $-NR^4R^5$-moiety,
one of the residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an $-SO_2-R^6$-moiety; or an $-NR^7R^8$-moiety, or $R^4$ and $R^5$, identical or different, represent a methyl group, an ethyl group, an n-propyl group, an isopropyl group, an n-butyl group, a sec-butyl group or a

tert-butyl group,
$R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups, and
$R^7$ and $R^8$, Identical or different, represent a hydrogen atom or a $C_{1-6}$ alkyl radical

optionally in form of a corresponding N-oxide thereof, or a corresponding salt thereof, or a corresponding solvate thereof.

**2.** The compound (R)-N-piperidinyl-5-(4-chlorophenyl)-1-(2,4-dichlorophenyl)-4,5-dihydro-1H-pyrazol-3-carboxamide according to claim 1 :

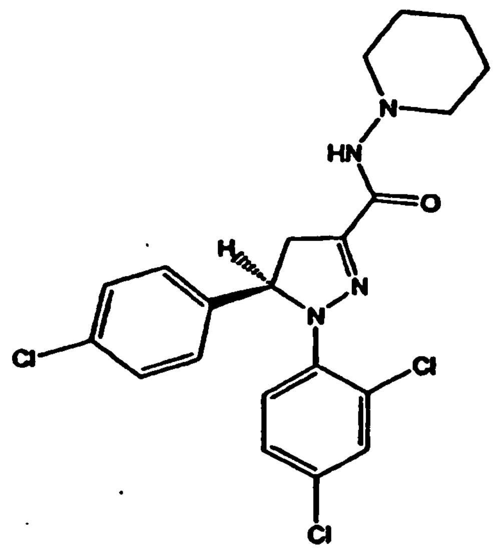

optionally In the form of a corresponding N-oxide, a corresponding salt or a corresponding solvate.

**3.** Process for the manufacture of substituted pyrazoline compounds of general formula I according to one or more of claims 1 to 2, **characterized in that** at least one compound of general formula IIa

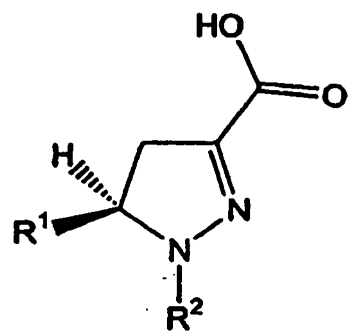

**(IIa)**

wherein $R^1$ and $R^2$ have the meaning according to one or more of claims 1-2, is optionally transferred under inert atmosphere to a compound of general formula (III) via reaction with an activating agent

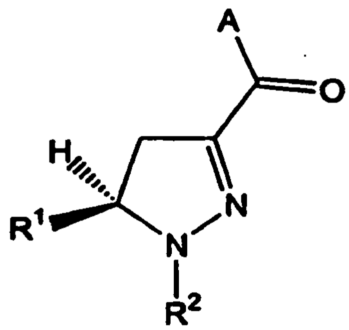

**(III)**

wherein the substituents $R^1$ and $R^2$ have the meaning given above and A represents a leaving group, preferably a chlorine atom, said compound being optionally isolated and/or optionally purified, and at least one compound of general formula (IIa) is reacted with a compound of general formula $R^3H$, wherein $R^3$ represents an $-NR^4R^5$-moiety, with $R^4$ and $R^5$ having the meaning according to one or more of claims 1-2 under inert atmosphere to yield a substituted pyrazoline compound of general formula I, wherein $R^3$ represents an $-NR^4R^5$-moiety,
or at least one compound of general formula (III) is reacted with a compound of the general formula $R^3H$, in which $R^3$ has the meaning according to one or more of claims 1-2 under inert atmosphere to yield a compound of general formula (I) according to one or more of claims 1-2, which is optionally isolated and/or optionally purified.

**4.** Process according to claim 3, wherein the compound IIa is obtained from a mixture comprising the enantiomers

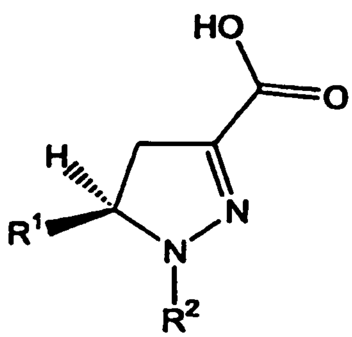

and

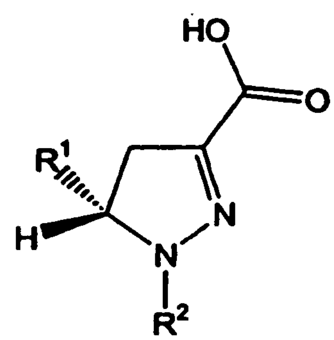

**(IIa)** **(IIb).**

**5.** Process according to claim 4, wherein the compound IIa is obtained from the mixture in form of an addition compound with a chiral base, preferably (+)-Cinchonine or R-(+)-1-Phenylethylamine, and preferably liberated from the addition compound.

**6.** Process according to claim 4 or 5, wherein the mixture comprising the enantiomers IIa and IIb is obtained by reacting at least one benzaldehyde compound of general formula IV

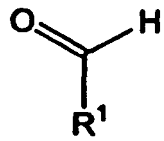

(IV)

wherein $R^1$ has the meaning according to one or more of claims 1-2, is reacted with a pyruvate compound of general formula (V)

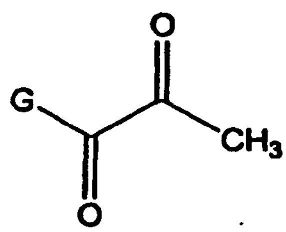

(V),

wherein G represents an OR group with R being a branched or unbranched $C_{1-6}$ alkyl radical or G represents an $O^-K$ group with K being a cation, to yield a compound of general formula (VI)

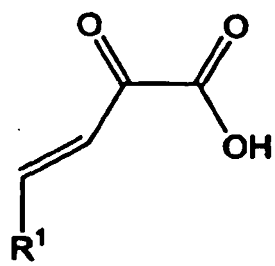

(VI)

wherein $R^1$ has the meaning given above, which is optionally isolated and/or optionally purified, and which is reacted with an optionally substituted phenyl hydrazine of general formula (VII)

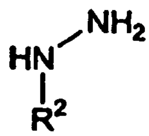

(VII)

or a corresponding salt thereof, wherein $R^2$ has the meaning according to one or more of claims 1-2, under inert atmosphere, to yield a mixture of compounds

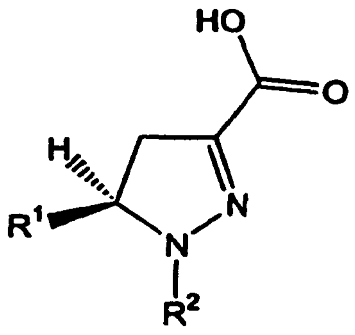

and

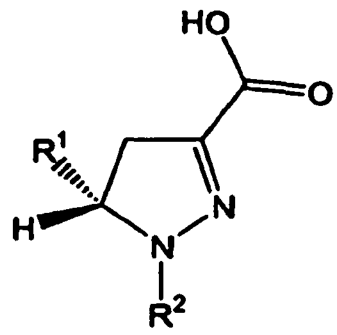

(IIa) (IIb).

7. Medicament comprising one or more substituted pyrazoline compounds of general formula I according to one or more of claims 1-2 and optionally one or more pharmaceutically acceptable excipients.

8. Medicament according to claim 7 for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

9. Medicament according to claim 7 or 8 for the prophylaxis and/or treatment of food intake disorders, preferably bulimia, anorexia, cachexia, obesity, type II diabetus mellitus (non-insuline dependent diabetes mellitus), more preferably obesity.

10. Medicament according to claim 7 or 8 for the prophylaxis and/or treatment of psychosis.

11. Medicament according claim 7 or 8 for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

12. Medicament according to claim 7 or 8 for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

13. Medicament according to claim 7 or 8 for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhoea, asthma, memory disorders, pruritus, pain, or for potentiation of the analgesic effect of narcotic and non-narcotic analgesics, or for influencing intestinal transit.

14. Use of at least one substituted pyrazoline compound according to one or more of claims 1-2 and optionally one or more pharmaceutically acceptable excipients, for the preparation of a medicament for the modulation of cannabinold-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**15.** Use of at least one substituted pyrazoline compound according to one or more of claims 1-2 and optionally one or more pharmaceutically acceptable excipients, for the preparation of a medicament for the prophylaxis and/or treatment of food intake disorders, preferably bulimia, anorexia, cachexia, obesity, type II diabetus mellitus (non-insuline dependent diabetes mellitus), more preferably obesity.

**16.** Use of at least one substituted pyrazoline compound according to one or more of claims 1-2 and optionally one or more pharmaceutically acceptable excipients, for the preparation of a medicament for the prophylaxis and/or treatment of psychosis.

**17.** Use of at least one substituted pyrazoline compound according to one or more of claims 1-2 and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**18.** Use of at least one substituted pyrazoline compound according to one or more of claims 1-2 and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**19.** Use of at least one substituted pyrazoline compound according to one or more of claims 1-2 and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, Immunologic disorders, sclerotic plaques, vomiting, diarrhoea, asthma, memory disorders, pruritus, pain, or for potentiation of the analgesic effect of narcotic and non-narcotic analgesics, or for influencing intestinal transit.

## Patentansprüche

**1.** Verbindungen der allgemeinen Formel I:

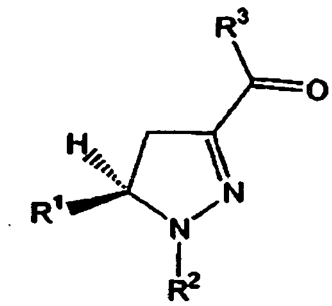

I

wobei

$R^1$ für eine Phenylgruppe steht, die gegebenenfalls durch einen oder mehrere Substituenten substituiert ist, die unanhängig voneinander ausgewählt sind aus der Gruppe, die aus Methyl, Ethyl, F, Cl, Br und $CF_3$ besteht,
$R^2$ für eine Phenylgruppe steht, die gegebenenfalls durch einen oder mehrere Substituenten substituiert ist, die unanhängig voneinander ausgewählt sind aus der Gruppe, die aus Methyl, Ethyl, F, Cl, Br und $CF_3$ besteht,
$R^3$ für eine Pyrrolidinyl-Gruppe, eine Piperidinyl-Gruppe oder eine Piperazinyl-Gruppe steht, wobei jede dieser Gruppen mit einer oder mehreren $C_{1-6}$-Alkylgruppen substituiert sein kann, oder $R^3$ für eine funktionelle $-NR^4R^5$-Gruppe steht,
einer von den Resten $R^4$ und $R^5$ für ein Wasserstoffatom steht und der andere von den Resten $R^4$ und $R^5$ für eine gegebenenfalls mindestens monosubstituierte Pyrrolidinyl-Gruppe; eine gegebenenfalls mindestens monosubstituierte Piperidinyl-Gruppe; eine gegebenenfalls mindestens monosubstituierte Piperazinyl-Gruppe; eine gegebenenfalls mindestens monosubstituierte Triazolyl-Gruppe; eine funktionelle $-SO_2-R^6$-Gruppe; oder eine funktionelle $-NR^7R^8$-Gruppe steht, oder $R^4$ und $R^5$ stehen identisch oder unterschiedlich für eine Methylgruppe, eine Ethylgruppe, eine n-Propylgruppe, eine Isopropylgruppe, eine n-Butylgruppe, eine sec-Butylgruppe oder eine tert-Butylgruppe,
$R^6$ für eine $C_{1-6}$-Alkylgruppe; für eine gesättigte, gegebenenfalls mindestens monosubstituierte cycloaliphatische Gruppe, die mit einem mono- oder polyzyklischen Ringsystem kondensiert sein kann; oder für eine Phenylgruppe steht, die gegebenenfalls mit einer oder mehreren $C_{1-6}$-Alkylgruppen substituiert ist, und
$R^7$ und $R^8$ identisch oder unterschiedlich für ein Wasserstoffatom oder ein $C_{1-6}$-Alkyl-Radikal stehen,
gegebenenfalls in der Form eines entsprechenden N-Oxids davon, oder eines entsprechenden Salzes davon oder eines entsprechenden Solvates davon.

**2.** Verbindung (R)-N-Piperidinyl-5-(4-chlor-phenyl)-1-(2,4-dichlorphenyl)-4,5-dihydro-1H-pyrazol-3-carboxamid nach Anspruch 1:

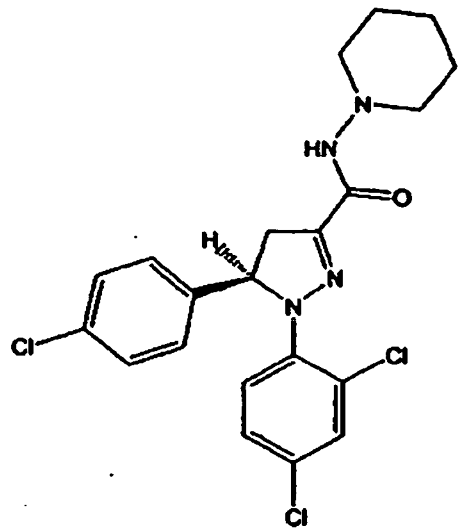

gegebenenfalls in der Form eines entsprechenden N-Oxids, eines entsprechenden Salzes oder eines entsprechenden Solvates.

**3.** Verfahren zur Herstellung von substituierten Pyrazolin-Verbindungen der allgemeinen Formel I nach einem oder mehreren der Ansprüche 1 bis 2, **dadurch gekennzeichnet, dass** mindestens eine Verbindung der allgemeinen Formel IIa,

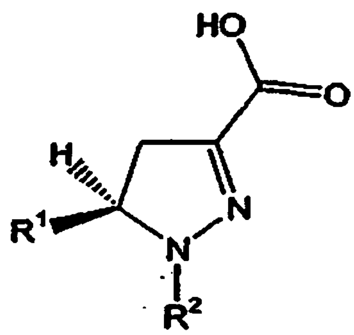

**(IIa)**

wobei $R^1$ und $R^2$ die Bedeutung nach einem oder mehreren der Ansprüche 1 bis 2 aufweisen, gegebenenfalls unter einer inerten Atmosphäre zu einer Verbindung der allgemeinen Formel (III)

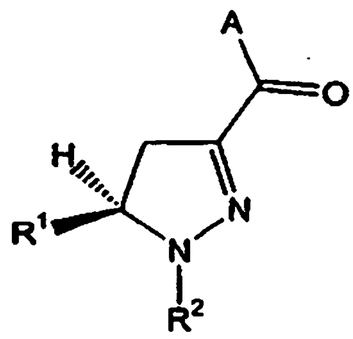

**(III)**

über die Umsetzung mit einem aktivierenden Agens überführt wird, wobei die Substituenten $R^1$ und $R^2$ dieselbe Bedeutung besitzen wie vorangehend angegeben und A für eine Fluchtgruppe, vorzugsweise ein Chloratom, steht, wobei die Verbindung gegebenenfalls isoliert und/oder gegebenenfalls gereinigt wird, und mindestens eine Verbindung der allgemeinen Formel (IIa) mit einer Verbindung der allgemeinen Formel $R^3H$ unter einer inerten Atmosphäre umgesetzt wird, wobei $R^3$ für eine funktionelle $-NR^4R^5$-Gruppe steht, wobei $R^4$ und $R^5$ die Bedeutung nach einem oder mehreren der Ansprüche 1 bis 2 besitzen, um eine substituierte Pyrazolin-Verbindung der allgemeinen Formel I zu erhalten, wobei $R^3$ für eine funktionelle $-NR^4R^5$-Gruppe steht,
oder mindestens eine Verbindung der allgemeinen Formel (III) mit einer Verbindung der allgemeinen Formel $R^3H$, in der $R^3$ die Bedeutung gemäß einem oder mehreren der Ansprüche 1 bis 2 besitzt, unter einer inerten Atmosphäre umgesetzt wird, um eine Verbindung der allgemeinen Formel (I) nach einem oder mehreren der Ansprüche 1 bis 2 zu ergeben, wobei die Verbindung gegebenenfalls isoliert und/oder gegebenenfalls gereinigt wird.

4. Verfahren nach Anspruch 3, wobei die Verbindung IIa aus einem Gemisch erhalten wird, das die Enantiomere

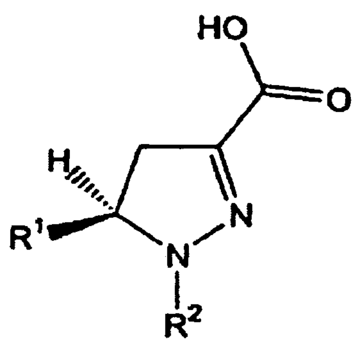

**(IIa)**

und

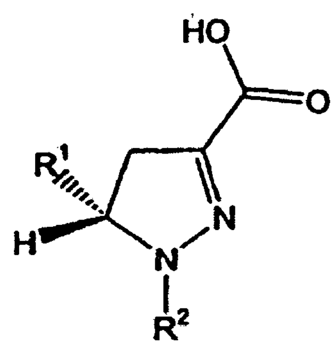

**(IIb).**

enthält.

5. Verfahren nach Anspruch 4, wobei die Verbindung IIa aus dem Gemisch in Form von einer Additionsverbindung mit einer chiralen Base erhalten wird, vorzugsweise (+)-Cinchonin oder R-{+)-1-Phenylethylamin, und vorzugsweise von der Additionsverbindung freigesetzt ist.

6. Verfahren nach Anspruch 4 oder 5, wobei das Gemisch, das die Enantiomere IIa und IIb enthält, durch Umsetzen von mindestens einer Benzaldehydverbindung der allgemeinen Formel IV,

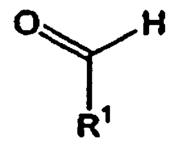

(IV)

wobei $R^1$ die Bedeutung nach einem oder mehreren der Ansprüche 1 bis 2 besitzt, mit einer Pyruvatverbindung der allgemeinen Formel (V)

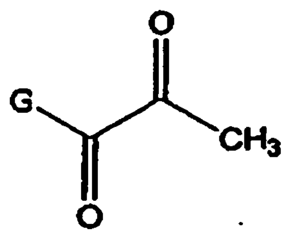

(V),

erhalten wird, wobei G für eine OR-Gruppe steht, wobei R ein verzweigtes oder unverzweigtes $C_{1-6}$-Alkylradikal ist, oder G steht für eine O-K-Gruppe, wobei K ein Kation ist, um eine Verbindung der allgemeinen Formel (VI)

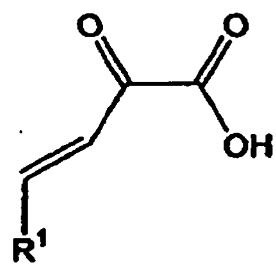

(VI)

zu ergeben, wobei $R^1$ die vorangehend angegebene Bedeutung besitzt, die gegebenenfalls isoliert und/oder gegebenenfalls gereinigt wird, und die mit einem gegebenenfalls substituierten Phenylhydrazin der allgemeinen Formel (VII)

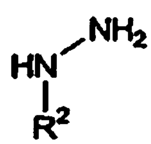

(VII)

oder einem entsprechenden Salz davon unter einer inerten Atmosphäre umgesetzt wird, wobei $R^2$ die Bedeutung nach einem oder mehreren der Ansprüche 1 bis 2 besitzt, um ein Gemisch der Verbindungen

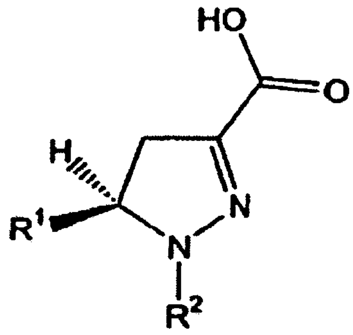 und 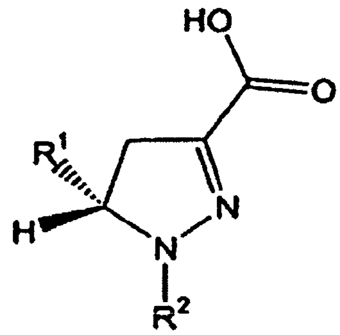

(IIa) (IIb).

zu ergeben.

**7.** Medikament, das eine oder mehrere der substituierten Pyrazolinverbindungen der allgemeinen Formel I nach einem oder mehreren der Ansprüche 1 bis 2 und gegebenenfalls einen oder mehrere pharmazeutisch verträgliche Hilfsstoffe enthält.

**8.** Medikament nach Anspruch 7 für die Modulation von Cannabinoid-Rezeptoren, vorzugsweise Cannabinoid-1-Rezeptoren (CB,-Rezeptoren) zur Vorbeugung und/oder zur Behandlung von Erkrankungen des Zentralnervensystems, Erkrankungen des Immunsystems, Erkrankungen des kardiovaskulären Systems, Erkrankungen des endokrinen Systems, Erkrankungen des Respirationssystems, Erkrankungen des Gastrointestinaltrakts oder Störungen der Fortpflanzungsfähigkeit.

**9.** Medikament nach Anspruch 7 oder 8 für die Vorbeugung und/oder die Behandlung von Störungen der Nahrungsaufnahme, vorzugsweise Bulimie, Anorexie, Kachexie, Fettleibigkeit, Typ-II Diabetes mellitus (nicht insulinabhängiger Diabetes mellitus), insbesondere bevorzugt Fettleibigkeit.

**10.** Medikament nach Anspruch 7 oder 8 für die Vorbeugung und/oder die Behandlung von psychotischen Störungen.

**11.** Medikament nach Anspruch 7 oder 8 für die Vorbeugung und/oder Behandlung von Alkoholmissbrauch und/oder Alkoholabhängigkeit, Nikotinmissbrauch und/oder Nikotinabhängigkeit, Drogenmissbrauch und/oder Drogenabhängigkeit und/oder Medikamentenmissbrauch und/oder Medikamentenabhängigkeit, vorzugsweise Drogenmissbrauch und/oder Drogenabhängigkeit und/oder Nikotinmissbrauch und/oder Nikotinabhängigkeit.

**12.** Medikament nach Anspruch 7 oder 8 für die Vorbeugung und/oder Behandlung von Krebs, vorzugsweise für die Vorbeugung und/oder Behandlung von einer oder mehreren Arten von Krebs, die ausgewählt sind aus der Gruppe, die aus Hirnkrebs, Knochenkrebs, Lippenkarzinom, Mundkrebs, Speiseröhrenkrebs, Magenkrebs, Leberkrebs, Blasenkrebs, Bauchspeicheldrüsenkrebs, Eierstockkrebs, Gebärmutterkrebs, Lungenkrebs, Brustkrebs, Hautkrebs, Dickdarmkrebs, Darmkrebs und Prostatakrebs besteht, insbesondere bevorzugt für die Vorbeugung und/oder Behandlung von einer oder mehreren Arten von Krebs, die ausgewählt sind aus der Gruppe, die aus Dickdarmkrebs, Darmkrebs und Prostatakrebs besteht.

**13.** Medikament nach Anspruch 7 oder 8 für die Vorbeugung und/oder Behandlung von einer oder mehreren Erkrankungen, die ausgewählt sind aus der Gruppe, die aus Knochenerkrankungen, vorzugsweise Osteoporose (z. B. Osteoporose, die mit einer genetischen Prädisposition, Sexualhormonmangel und Altern verbunden ist), mit Krebs verbundenen Knochenkrankheiten oder Morbus Paget; Schizophrenie, Angst, Depression, Epilepsie, neurodegenerativen Erkrankungen, zerebellären Störungen, spinozerebellären Störungen, kognitiven Störungen, Schädeltrauma, Kopftrauma, Schlaganfall, Panikattacken, peripherer Neuropathie, Glaukom, Migräne, Morbus Parkinson, Morbus Huntington, Morbus Alzheimer, Raynaud-Krankheit, mit Zittern einhergehenden Erkrankungen, zwangsneurotischen Erkrankungen, Altersdemenz, Erkrankungen der Thymusdrüse, tardiver Dyskinesie, bipolaren Störungen, medikamenteninduzierten Bewegungsstörungen, Dystonie, endotoxischem Schock, hämorrhagischem Schock, Hypotonie, Schlaflosigkeit, immunologischen Erkrankungen, sklerotischen Plaques, Erbrechen, Durchfall, Asthma, Gedächtnisstörungen, Juckreiz, Schmerz besteht, oder zur Potenzierung der schmerzlindernden Wirkung von narkotischen und nichtnarkotischen Schmerzmitteln, oder zur Beeinflussung der Darmpassage.

**14.** Verwendung von mindestens einer substituierten Pyrazolin-Verbindung nach einem oder mehreren der Ansprüche 1 bis 2 und gegebenenfalls von einem oder mehreren pharmazeutisch verträglichen Hilfsstoffen, zur Herstellung eines Medikaments zur Modulation von Cannabinoid-Rezeptoren, vorzugsweise der Cannabinoid-1-Rezeptoren ($CB_1$-Rezeptoren), für die Vorbeugung und/oder Behandlung von Erkrankungen des Zentralnervensystems, Erkrankungen des Immunsystems, Erkrankungen des kardiovaskulären Systems, Erkrankungen des endokrinen Systems, Erkrankungen des Respirationssystems, Erkrankungen des Gastrointestinaltrakts oder Störungen der Fortpflanzungsfähigkeit.

**15.** Verwendung von mindestens einer substituierten Pyrazolin-Verbindung nach einem oder mehreren der Ansprüche 1 bis 2 und gegebenenfalls von einem oder mehreren pharmazeutisch verträglichen Hilfsstoffen, zur Herstellung eines Medikaments für die Vorbeugung und/oder Behandlung von Störungen der Nahrungsaufnahme, vorzugsweise Bulimie, Anorexie, Kachexie, Fettleibigkeit, Typ-II Diabetes mellitus (nicht insulinabhängiger Diabetes mellitus), insbesondere bevorzugt Fettleibigkeit.

**16.** Verwendung von mindestens einer substituierten Pyrazolin-Verbindung nach einem oder mehreren der Ansprüche 1 bis 2 und gegebenenfalls von einem oder mehreren pharmazeutisch verträglichen Hilfsstoffen, zur Herstellung eines Medikaments für die Vorbeugung und/oder die Behandlung von psychotischen Störungen.

**17.** Verwendung von mindestens einer substituierten Pyrazolin-Verbindung nach einem oder mehreren der Ansprüche 1 bis 2 und gegebenenfalls von einem oder mehreren pharmazeutisch verträglichen Hilfsstoffen, zur Herstellung eines Medikaments für die Vorbeugung und/oder die Behandlung von Alkoholmissbrauch und/oder Alkoholabhängigkeit, Nikotinmissbrauch und/oder Nikotinabhängigkeit, Drogenmissbrauch und/oder Drogenabhängigkeit und/oder Medikamentenmissbrauch und/oder Medikamentenabhängigkeit, vorzugsweise Drogenmissbrauch und/oder Drogenabhängigkeit und/oder Nikotinmissbrauch und/oder Nikotinabhängigkeit.

**18.** Verwendung von mindestens einer substituierten Pyrazolin-Verbindung nach einem oder mehreren der Ansprüche 1 bis 2 und gegebenenfalls von einem oder mehreren pharmazeutisch verträglichen Hilfsstoffen, zur Herstellung eines Medikaments für die Vorbeugung und/oder Behandlung von Krebs, vorzugsweise für die Vorbeugung und/oder Behandlung von einer oder mehreren Arten von Krebs, die ausgewählt sind aus der Gruppe, die aus Hirnkrebs, Knochenkrebs, Lippenkarzinom, Mundkrebs, Speiseröhrenkrebs, Magenkrebs, Leberkrebs, Blasenkrebs, Bauchspeicheldrüsenkrebs, Eierstockkrebs, Gebärmutterkrebs, Lungenkrebs, Brustkrebs, Hautkrebs, Dickdarmkrebs, Darmkrebs und Prostatakrebs besteht, insbesondere bevorzugt für die Vorbeugung und/oder Behandlung von einer oder mehreren Arten von Krebs, die ausgewählt sind aus der Gruppe, die aus Dickdarmkrebs, Darmkrebs und Prostatakrebs besteht.

**19.** Verwendung von mindestens einer substituierten Pyrazolin-Verbindung nach einem oder mehreren der Ansprüche 1 bis 2 und gegebenenfalls von einem oder mehreren pharmazeutisch verträglichen Hilfsstoffen, zur Herstellung eines Medikaments für die Vorbeugung und/oder die Behandlung von einer oder mehreren Erkrankungen, die ausgewählt sind aus der Gruppe, die aus Knochenerkrankungen, vorzugsweise Osteoporose (z. B. Osteoporose, die mit einer genetischen Prädisposition, Sexualhormonmangel und Altern verbunden ist), mit Krebs verbundenen Knochenkrankheiten oder Morbus Paget; Schizophrenie, Angst, Depression, Epilepsie, neurodegenerativen Erkrankungen, zerebellären Störungen, spinozerebellären Störungen, kognitiver Störungen, Schädeltrauma, Kopftrauma, Schlaganfall, Panikattacken, peripherer Neuropathie, Glaukom, Migräne, Morbus Parkinson, Morbus Huntington, Morbus Alzheimer, Raynaud-Krankheit, mit Zittern einhergehenden Erkrankungen, zwangsneurotischen Erkrankungen, Altersdemenz, Erkrankungen der Thymusdrüse, tardiver Dyskinesie, bipolaren Störungen, medikamenteninduzierten Bewegungsstörungen, Dystonie, endotoxischem Schock, hämorrhagischem Schock, Hypotonie, Schlaflosigkeit, immunologischen Erkrankungen, sklerotischen Plaques, Erbrechen, Durchfall, Asthma, Gedächtnisstörungen, Juckreiz, Schmerz besteht, oder zur Potenzierung der schmerzlindernden Wirkung von narkotischen und nichtnarkotischen Schmerzmitteln, oder zur Beeinflussung der Darmpassage.

**Revendications**

**1.** Composés de formule générale (I)

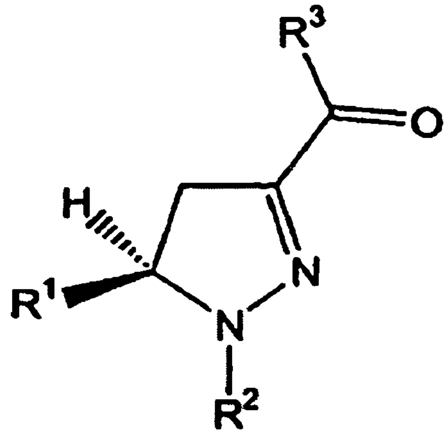

I

dans laquelle

$R^1$ représente un groupe phényle, qui est facultativement substitué par un ou plusieurs substituants indépendamment choisis dans le groupe constitué par les groupes méthyle, éthyle, F, Cl, Br et $CF_3$,
$R^2$ représente un groupe phényle, qui est facultativement substitué par un ou plusieurs substituants indépendamment choisis dans le groupe constitué par les groupes méthyle, éthyle, F, Cl, Br et $CF_3$,
$R^3$ représente un groupe pyrrolidinyle, un groupe pipéridinyle ou un groupe pipérazinyle, chacun de ces groupes pouvant ainsi être substitué par un ou plusieurs groupes alkyles en $C_{1-6}$, ou $R^3$ représente une fraction $-NR^4R^5-$, l'un des résidus $R^4$ et $R^5$ représente un atome d'hydrogène et l'autre de ces résidus $R^4$ et $R^5$ représente un groupe pyrrolidinyle facultativement au moins monosubstitué ; un groupe pipéridinyle facultativement au moins monosubstitué ; un groupe pipérazinyle facultativement au moins monosubstitué ; un groupe triazolyle facultativement au moins monosubstitué ; une fraction $-SO_2-R^6-$ ; ou une fraction $-NR^7R^8-$, ou $R^4$ et $R^5$, identiques ou différents, représentent un groupe méthyle, un groupe éthyle, un groupe n-propyle, un groupe isopropyle, un groupe n-butyle, un groupe sec-butyle ou un groupe tert-butyle,
$R^6$ représente un groupe alkyle en $C_{1-6}$ ; un groupe cycloaliphatique saturé, facultativement au moins monosubstitué, qui peut être condensé avec un système cyclique mono- ou polycyclique ; ou un groupe phényle, qui est facultativement substitué par un ou plusieurs groupes alkyle en $C_{1-6}$, et
$R^7$ et $R^8$, identiques ou différents, représentent un atome d'hydrogène ou un radical alkyle en $C_{1-6}$

facultativement sous la forme d'un N-oxyde correspondant de ceux-ci, ou d'un sel correspondant de ceux-ci, ou d'un solvate correspondant de ceux-ci.

**2.** Composé (R)-N-pipéridinyl-5-(4-chloro-phényl)-1-(2,4-dichlorophényl)-4,5-dihydro-1H-pyrazol-3-carboxamide selon la revendication 1 :

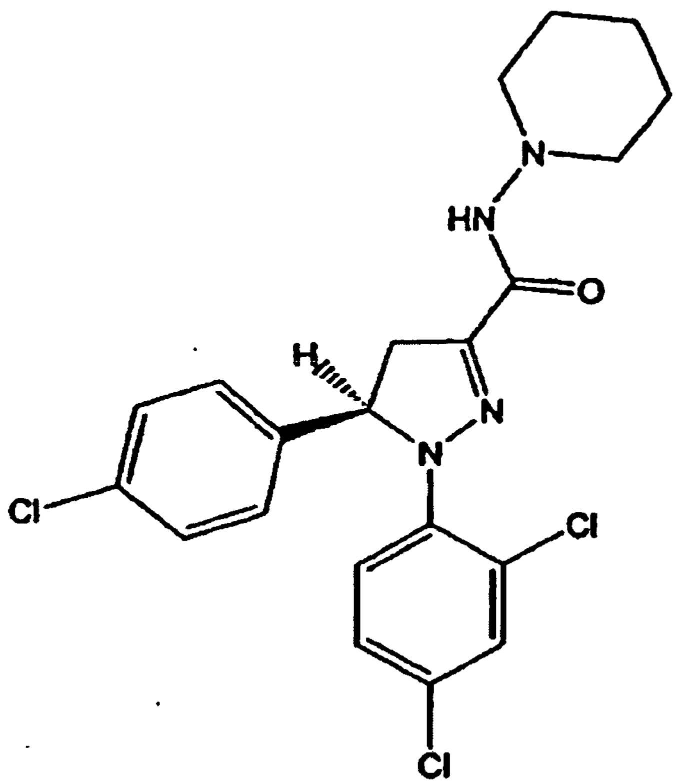

facultativement sous la forme d'un N-oxyde correspondant, d'un sel correspondant ou d'un solvate correspondant.

**3.** Procédé de fabrication des composés pyrazoline substitués de formule générale I selon une ou plus des revendications 1 et 2, **caractérisé en ce qu'**au moins un composé de formule générale IIa,

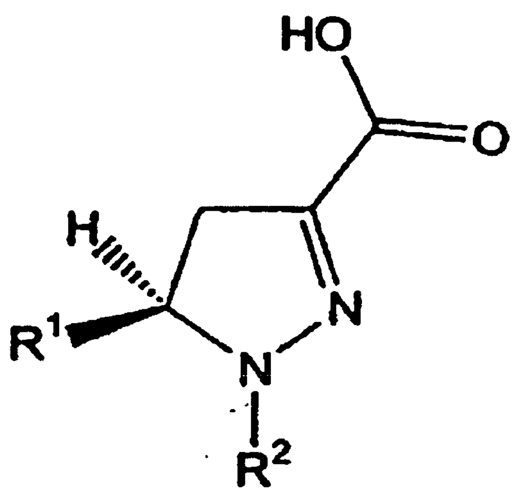

**(IIa)**

dans laquelle $R^1$ et $R^2$ ont la signification selon une ou plus des revendications 1 et 2, est facultativement transféré

sous une atmosphère inerte à un composé de formule générale (III) via une réaction avec un agent d'activation

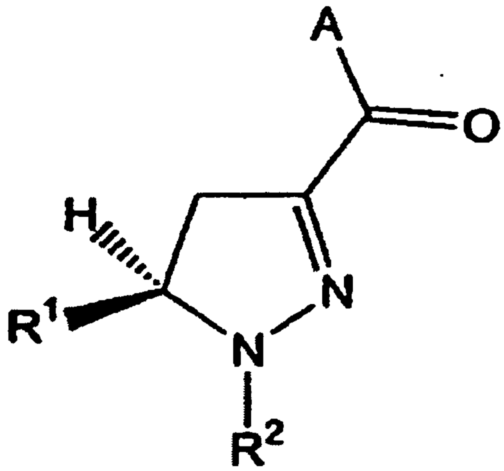

(III)

les substituants $R^1$ et $R^2$ ayant la signification proposée ci-dessus et A représentant un groupe partant, de préférence un atome de chlore, ledit composé étant
facultativement isolé et/ou facultativement purifié, et au moins un composé de formule générale (IIa) réagit avec un composé de formule générale $R^3H$, dans laquelle $R^3$ représente une fraction $-NR^4R^5-$, $R^4$ et $R^5$ ayant la signification selon une ou plus des revendications 1 et 2, sous une atmosphère inerte pour donner un composé pyrazoline substitué de formule générale I, dans laquelle $R^3$ représente une fraction $-NR^4R^5-$,
ou au moins un composé de formule générale (III) réagit avec un composé de formule générale $R^3H$, dans laquelle $R^3$ a la signification selon une ou plus des revendications 1 et 2, sous une atmosphère inerte pour donner un composé de formule générale (I) selon une ou plus des revendications 1 et 2, qui est facultativement isolé et/ou facultativement purifié.

4. Procédé selon la revendication 3, le composé IIa étant obtenu d'un mélange comprenant les énantiomères

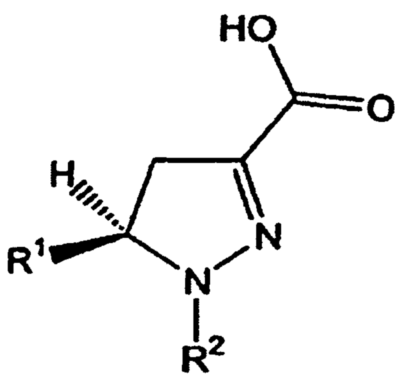

et

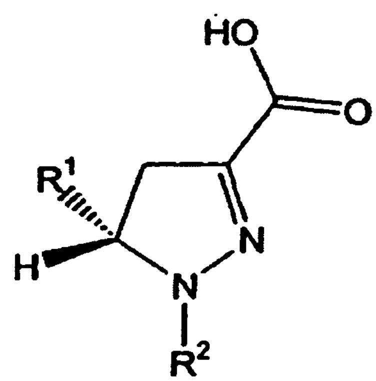

(IIa) (IIb).

5. Procédé selon la revendication 4, le composé IIa étant obtenu du mélange sous la forme d'un composé d'addition avec une base chirale, de préférence la (+)-cinchonine ou la R-(+)-1-phényléthylamine, et de préférence libéré du

composé d'addition.

**6.** Procédé selon la revendication 4 ou 5, le mélange comprenant les énantiomères IIa et IIb étant obtenu par réaction d'au moins un composé benzaldéhyde de formule générale IV

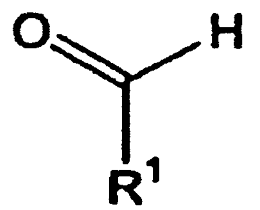

(IV)

dans laquelle $R^1$ a la signification selon une ou plus des revendications 1 et 2, avec un composé pyruvate de formule générale (V)

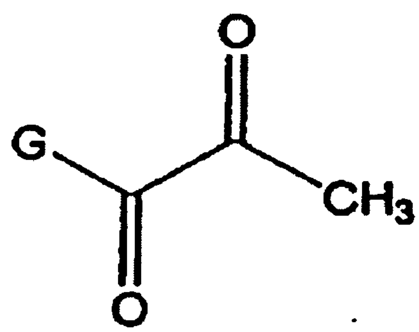

(V),

dans laquelle G représente un groupe OR, R étant un radical alkyle en $C_{1-6}$ ramifié ou non ramifié ou G représente un groupe $O^{-}K$, K étant un cation, pour donner un composé de formule générale (VI)

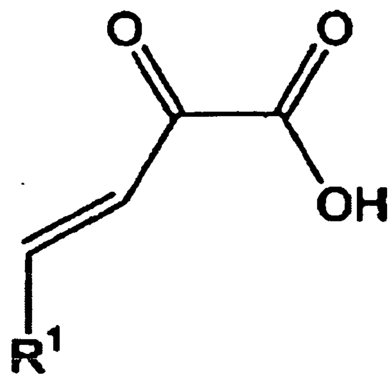

(VI)

dans laquelle $R^1$ a la signification proposée ci-dessus, qui est facultativement isolé et/ou facultativement purifié, et qui réagit avec une phényl hydrazine facultativement substituée de formule générale (VII)

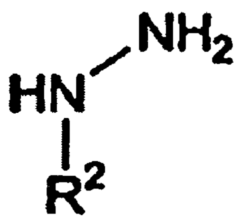

(VII)

ou un sel correspondant de celle-ci, dans laquelle $R^2$ a la signification selon une ou plus des revendications 1 et 2, sous une atmosphère inerte, pour donner un mélange des composés

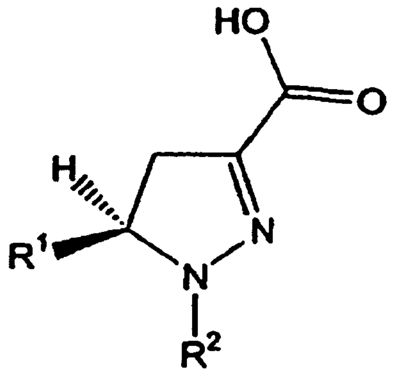

et

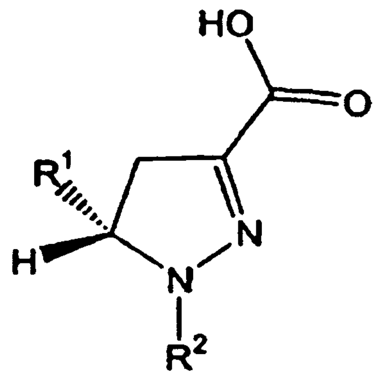

(IIa) (IIb).

7. Médicament comprenant un ou plusieurs composés pyrazoline substitués de formule générale I selon un ou plus des revendications 1 et 2 et facultativement un ou plusieurs excipients pharmaceutiquement acceptables.

8. Médicament selon la revendication 7, destiné à la modulation des récepteurs cannabinoïdes, de préférence des récepteurs cannabinoïdes 1 ($CB_1$), pour la prophylaxie et/ou le traitement de troubles du système nerveux central, de troubles du système immunitaire, de troubles du système cardiovasculaire, de troubles du système endocrinien, de troubles du système respiratoire, de troubles du tractus gastrointestinal ou de troubles de la reproduction.

9. Médicament selon la revendication 7 ou 8, destiné à la prophylaxie et/ou au traitement de troubles de l'alimentation, de préférence de la boulimie, de l'anorexie, de la cachexie, de l'obésité, du diabète sucré de type II (diabète non insulinodépendant) et de préférence l'obésité.

10. Médicament selon la revendication 7 ou 8, destiné à la prophylaxie et/ou au traitement de la psychose.

11. Médicament selon la revendication 7 ou 8, destiné au traitement de l'abus de l'alcool et/ou au traitement de l'alcoolisme, du tabagisme, de la toxicomanie et/ou de la toxicomanie aux médicaments, de préférence de la toxicomanie et/ou du tabagisme.

12. Médicament selon la revendication 7 ou 8, destiné à la prophylaxie et/ou au traitement du cancer, de préférence à la prophylaxie et/ou au traitement d'un ou de plusieurs types de cancers choisis dans le groupe constitué par le cancer du cerveau, le cancer des os, le cancer de la lèvre, le cancer de la bouche, le cancer de l'oesophage, le cancer de l'estomac, le cancer du foie, le cancer de la vessie, le cancer du pancréas, le cancer des ovaires, le cancer du col de l'utérus, le cancer du poumon, le cancer du sein, le cancer de la peau, le cancer du côlon, la cancer de l'intestin et le cancer de la prostate, de préférence à la prophylaxie et/ou au traitement d'un ou de plusieurs types de cancers choisis dans le groupe constitué par le cancer du côlon, la cancer de l'intestin et le cancer de la prostate.

13. Médicament selon la revendication 7 ou 8, destiné à la prophylaxie et/ou au traitement d'un ou de plusieurs troubles choisis dans le groupe constitué par les troubles osseux, de préférence l'ostéoporose (par exemple, l'ostéoporose associée à une prédisposition génétique, un déficit en hormones sexuelles, ou le vieillissement), une maladie osseuse liée à un cancer ou la maladie de Paget des os ; la schizophrénie, l'anxiété, la dépression, l'épilepsie, les troubles neurodégénératifs, les troubles cérébelleux, les troubles spino-cérébelleux, les troubles cognitifs, un traumatisme crânien, un accident vasculaire cérébral, les crises de panique, une neuropathie périphérique, le glaucome, la migraine, la maladie de Parkinson, la chorée d'Huntington, la maladie d'Alzheimer, la maladie de Raynaud, les tremblements, les troubles compulsifs, la démence sénile, les troubles thymiques, la dyskinésie tardive, les psychoses maniacodépressives, les troubles moteurs induits par des médicaments, la dystonie, un choc endotoxémique, un choc hémorragique, l'hypotension, l'insomnie, les troubles immunologiques, les plaques sclérotiques, les vomissements, les diarrhées, l'asthme, les troubles de la mémoire, le prurit, la douleur, ou destiné à la potentialisation de l'effet analgésique des analgésiques narcotiques et non narcotiques, ou destiné à influencer le transit intestinal.

**14.** Utilisation d'au moins un composé pyrazoline substitué selon une ou plus des revendications 1 à 2, et facultativement un ou plusieurs excipients pharmaceutiquement acceptables, pour la préparation d'un médicament destiné à la modulation des récepteurs cannabinoïdes, de préférence des récepteurs cannabinoïdes 1 ($CB_1$), pour la prophylaxie et/ou le traitement de troubles du système nerveux central, de troubles du système immunitaire, de troubles du système cardiovasculaire, de troubles du système endocrinien, de troubles du système respiratoire, de troubles du tractus gastrointestinal ou de troubles de la reproduction.

**15.** Utilisation d'au moins un composé pyrazoline substitué selon une ou plus des revendications 1 à 2, et facultativement d'un ou de plusieurs excipients pharmaceutiquement acceptables, pour la préparation d'un médicament destiné à la prophylaxie et/ou au traitement de trouble de l'alimentation, de préférence de la boulimie, de l'anorexie, de la cachexie, de l'obésité, du diabète sucré de type II (diabète non insulinodépendant) et de préférence l'obésité.

**16.** Utilisation d'au moins un composé pyrazoline substitué selon une ou plus des revendications 1 et 2, et facultativement d'un ou de plusieurs excipients pharmaceutiquement acceptables, pour la préparation d'un médicament destiné à la prophylaxie et/ou au traitement de la psychose.

**17.** Utilisation d'au moins un composé pyrazoline substitué selon une ou plus des revendications 1 à 2, et facultativement d'un ou de plusieurs excipients pharmaceutiquement acceptables, pour la préparation d'un médicament destiné à la prophylaxie et/ou au traitement de l'abus de l'alcool et/ou au traitement de l'alcoolisme,du tabagisme, de la toxicomanie et/ou de la toxicomanie aux médicaments, de préférence de la toxicomanie et/ou du tabagisme.

**18.** Utilisation d'au moins un composé pyrazoline substitué selon une ou plus des revendications 1 à 2, et facultativement d'un ou de plusieurs excipients pharmaceutiquement acceptables, pour la préparation d'un médicament destiné à la prophylaxie et/ou au traitement du cancer, de préférence à la prophylaxie et/ou au traitement d'un ou de plusieurs types de cancers choisis dans le groupe constitué par le cancer du cerveau, le cancer des os, le cancer de la lèvre, le cancer de la bouche, le cancer de l'oesophage, le cancer de l'estomac, le cancer du foie, le cancer de la vessie, le cancer du pancréas, le cancer des ovaires, le cancer du col de l'utérus, le cancer du poumon, le cancer du sein, le cancer de la peau, le cancer du côlon, la cancer de l'intestin et le cancer de la prostate, de préférence à la prophylaxie et/ou au traitement d'un ou de plusieurs types de cancers choisis dans le groupe constitué par le cancer du côlon, la cancer de l'intestin et le cancer de la prostate.

**19.** Utilisation d'au moins un composé pyrazoline substitué selon une ou plus des revendications 1 à 2, et facultativement d'un ou de plusieurs excipients pharmaceutiquement acceptables, pour la préparation d'un médicament destiné à la prophylaxie et/ou au traitement d'un ou de plusieurs troubles choisis dans le groupe constitué par les troubles osseux, de préférence l'ostéoporose (par exemple, l'ostéoporose associée à une prédisposition génétique, un déficit en hormones sexuelles, ou le vieillissement), une maladie osseuse liée à un cancer ou la maladie de Paget des os ; la schizophrénie, l'anxiété, la dépression, l'épilepsie, les troubles neurodégénératifs, les troubles cérébelleux, les troubles spino-cérébelleux, les troubles cognitifs, un traumatisme crânien, un accident vasculaire cérébral, les crises de panique, une neuropathie périphérique, le glaucome, la migraine, la maladie de Parkinson, la chorée d'Huntington, la maladie d'Alzheimer, la maladie de Raynaud, les tremblements, les troubles compulsifs, la démence sénile, les troubles thymiques, la dyskinésie tardive, la psychose maniacodépressive, les troubles moteurs induits par des médicaments, la dystonie, un choc endotoxémique, un choc hémorragique, l'hypotension, l'insomnie, les troubles immunologiques, les plaques sclérotiques, les vomissements, les diarrhées, l'asthme, les troubles de la mémoire, le prurit, la douleur, ou la potentialisation de l'effet analgésique des analgésiques narcotiques et non narcotiques, ou destiné à influencer le transit intestinal.

Figure 1:

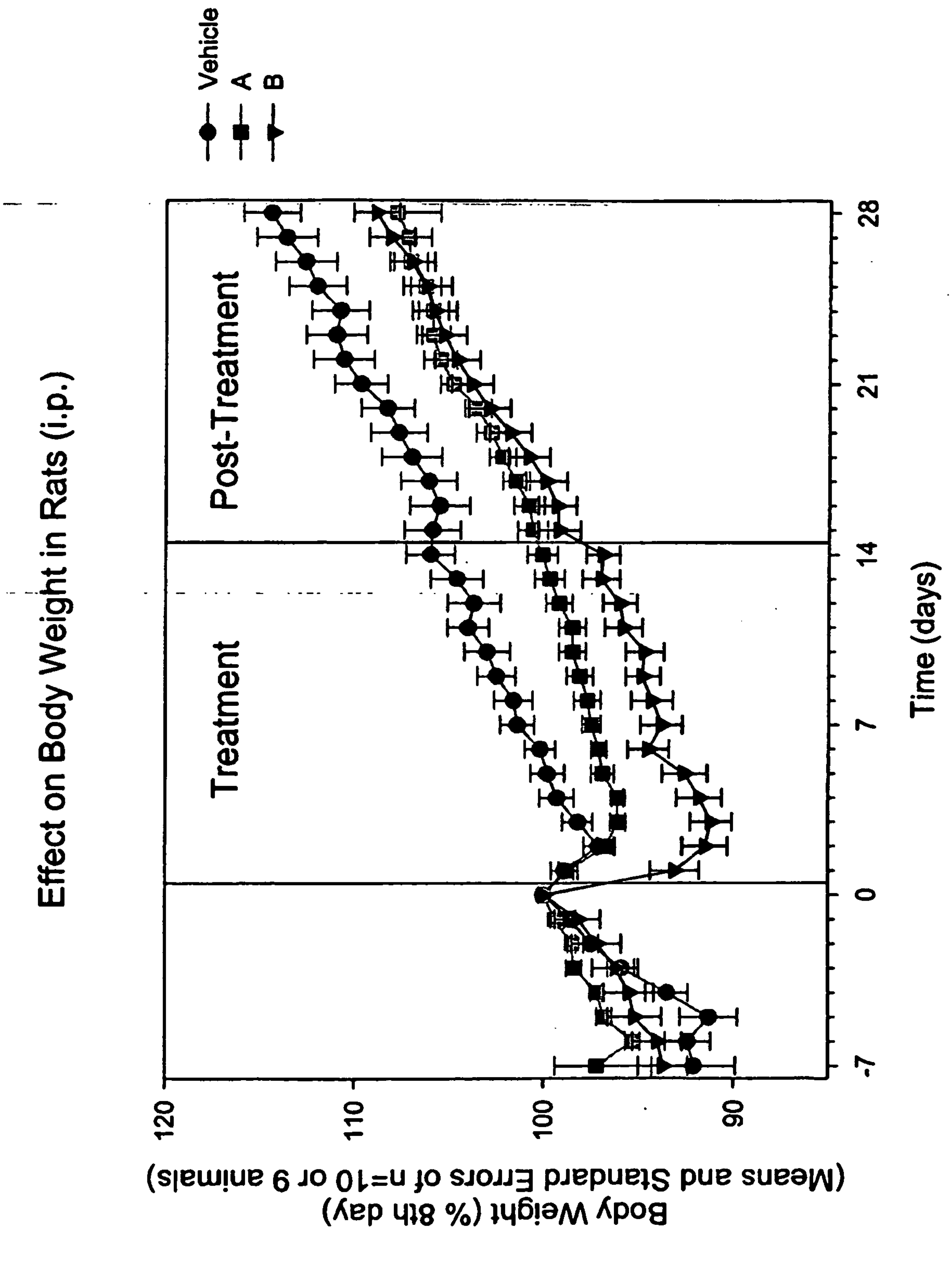
Effect on Body Weight in Rats (i.p.)
Treatment
Post-Treatment
Vehicle
A
B
Body Weight (% 8th day)
(Means and Standard Errors of n=10 or 9 animals)
120
110
100
90
-7
0
7
14
21
28
Time (days)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Hollister.** *Pharm. Rev.,* 1986, vol. 38, 1-20
- **Reny ; Singha.** *Prog. Drug. Res.,* 1991, vol. 36, 71-114
- **Consroe ; Sandyk.** Marijuana/Cannabinoids, Neurobiology and Neurophysiology. CRC Press, 1992, vol. 459
- *Synthetic communications,* 1996, vol. 26 (11), 2229-33
- *Synlett,* 2001, vol. 1, 147-149
- **Pascual, A.** *J. Prakt Chem.,* 1999, vol. 341 (7), 695-700
- **Lin, S. et al.** *Heterocycles,* 2001, vol. 55 (2), 265-277
- **Rao, P. et al.** *J. Org. Chem.,* 2000, vol. 65 (22), 7323-7344
- **Pearson D.E ; Buehler, C.A.** *Synthesis,* 1972, 533-542
- **Krogsgaard-Larsen et al.** Textbook of Drugdesign and Discovery. Taylor & Francis, April 2002
- **Ruth A. Ross ; Heather C. Brockie et al.** Agonist-inverse agonist characterization at CB1 and CB2 cannabinoid receptors of L-759633, L759656 and AM630. *British Journal of Pharmacology,* 1999, vol. 126, 665-672
- **Govaerts et al.** *Eur J Pharmac Sci,* 2004, vol. 23, 233-243
- **A. C. Howlett et al.** International Union of Pharmacology XXVII. *Classification of Cannabinoid Receptors, Pharmacol Rev,* 2002, vol. 54, 161-202
- **David R. Compton et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **Woolfe D. et al.** The evaluation of analgesic action of pethidine hydrochloride (Demerol). *J. Pharmacol. Exp. Ther.,* 1944, vol. 80, 300-307
- **Desmet L. K. C. et al.** Anticonvulsive properties of Cinarizine and Flunarizine in Rats and Mice. *Arzneim. -Forsch. (Frug Res),* 1975, vol. 25, 9
- **David R. Compton et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **Alpermann H. G. et al.** Pharmacological effects of Hoe 249: A new potential antidepressant. *Drugs Dev. Res.,* 1992, vol. 25, 267-282
- **G. Colombo et al.** Appetite Suppression and Weight Loss after the Cannabinoid Antagonist SR 141716. *Life Sciences,* 1998, vol. 63 (8), 113-117
- **E.T. Tzavara et al.** The CB1 receptor antagonist SR141716A selectively increases monoaminergic neurotransmission in the medial prefrontal cortex: implications for therapeutic actions. *Br. J. Pharmacol.,* 2003, vol. 138 (4), 544-53
- **Lumeng, L et al.** Different sensitivities to ethanol in alcohol-preferring and-nonpreferring rats. *Pharmacol, Biochem Behav.,* 1982, vol. 16, 125-130
- **Middaugh et al.** Ethanol Consumption by C57BU6 Mice : Influence of Gender and Procedural Variables. *Alcohol,* 1999, vol. 17 (3), 175-183
- **Le et al.** Alcohol Consumption by C57BL/6, BALA/c, and DBA/2 Mice in a Limited AccessParadigm. *PharmacologyBiochemistry and Behavior,* 1994, vol. 47, 375-378
- **Vastola et al.** *Physiol. Behav.,* 2002, vol. 77, 107-114
- **Brower et al.** *Brain Res.,* 2002, vol. 930, 12-20
- **Porsolt et al.** *Nature,* 1977, vol. 266, 730-732
- **Stem et al.** *Psychopharmacology,* 1985, vol. 85, 367-370
- **Swerdlow ; Geyer.** Schizophrenia Bulletin. 1998, vol. 24, 285-301
- **Pellow ; File.** *Pharm. Biochem. Behav.,* 1986, vol. 24, 525-529
- **Andersson et al.** *J. Urol.,* February 1999, vol. 161, 1707-17
- **Hale et al.** *Int. J. Impot. Res. 15,* 2003, vol. 5, 75-79